(19) Europäisches Patentamt
European Patent Office
Office européen des brevets

(11) **EP 2 592 141 B1**

(12) **EUROPEAN PATENT SPECIFICATION**

(45) Date of publication and mention
of the grant of the patent:
**30.12.2015 Bulletin 2015/53**

(51) Int Cl.:
*C12N 15/00* (2006.01)    *A01K 67/027* (2006.01)
*C12N 5/09* (2010.01)    *C12N 15/09* (2006.01)
*C12Q 1/02* (2006.01)

(21) Application number: **11803711.8**

(22) Date of filing: **07.07.2011**

(86) International application number:
**PCT/JP2011/066029**

(87) International publication number:
**WO 2012/005383 (12.01.2012 Gazette 2012/02)**

(54) **METHOD FOR PRODUCTION OF TUMOR CELLS FROM NORMAL MAMMARY EPITHELIAL CELLS**

VERFAHREN ZUR HERSTELLUNG VON TUMORZELLEN AUS NORMALEN SÄUGETIER-EPITHELZELLEN

PROCÉDÉ DE PRODUCTION DE CELLULES TUMORALES À PARTIR DE CELLULES ÉPITHÉLIALES MAMMAIRES NORMALES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **07.07.2010 JP 2010154744**

(43) Date of publication of application:
**15.05.2013 Bulletin 2013/20**

(73) Proprietor: **Eisai R&D Management Co., Ltd. Tokyo 112-8088 (JP)**

(72) Inventors:
• **AKAGI, Tsuyoshi**
**Kobe-shi, Hyogo 650-0047 (JP)**
• **SUKEZANE, Taiko**
**Kobe-shi, Hyogo 650-0047 (JP)**

(74) Representative: **Woods, Geoffrey Corlett**
**J A Kemp**
**14 South Square**
**Gray's Inn**
**London WC1R 5JJ (GB)**

(56) References cited:
**JP-A- 2008 522 626    JP-A- 2008 535 477
JP-A- 2010 514 759    US-A1- 2006 222 590**

• **MOISSOGLU KONSTADINOS ET AL: "v-Src rescues actin-based cytoskeletal architecture and cell motility and induces enhanced anchorage independence during oncogenic transformation of focal adhesion kinase-null fibroblasts.", JOURNAL OF BIOLOGICAL CHEMISTRY, vol. 278, no. 48, 28 November 2003 (2003-11-28), pages 47946-47959, XP002715360, ISSN: 0021-9258**
• **MACAULEY A ET AL: "COOPERATIVE TRANSFORMING ACTIVITIES OF RAS MYC AND SRC VIRAL ONCOGENES IN NONESTABLISHED RAT ADRENOCORTICAL CELLS", JOURNAL OF VIROLOGY, vol. 62, no. 12, 1988, pages 4712-4721, XP002715361, ISSN: 0022-538X**
• **LERMA ENRIQUE ET AL: "Immunohistochemical heterogeneity of breast carcinomas negative for estrogen receptors, progesterone receptors and Her2/neu (basal-like breast carcinomas)", MODERN PATHOLOGY, NATURE PUBLISHING GROUP, US, vol. 20, no. 11, 1 November 2007 (2007-11-01), pages 1200-1207, XP002536254, ISSN: 0893-3952, DOI: 10.1038/MODPATHOL. 3800961 [retrieved on 2007-09-21]**
• **TRTKOVA, K ET AL.: 'Deletions in the DNA-binding domain of the TP53 gene in v-src-transformed chicken cells' IN VITRO CELL.DEV.BIOL.-AMINAL vol. 40, 2004, pages 285 - 292, XP055085746**

- SHAULIAN, E. ET AL.: 'Identification of a minimal transforming domain of p53: Negative dominance through abrogation of sequence- specific DNA binding' MOL.CELL.BIOL. vol. 12, no. 12, 1992, pages 5581 - 5592, XP000676314
- KENDALL, S.D. ET AL.: 'A network of genetic events sufficient to convert normal human cells to a tumorigenic state' CANCER RES. vol. 65, 2005, pages 9824 - 9828, XP055098840
- YASUSHI YATABE: 'Kokei Shuyo' JAPANESE JOURNAL OF CLINICAL MEDICINE vol. 63, no. 3, 2005, pages 434 - 440
- RIDRIGUEZ-PUEBLA, M.L. ET AL.: 'cdk4 Deficiency inhibits skin tumor development but dose not affect normal keratinocyte proliferation' AM.J.PATHOL. vol. 161, no. 2, 2002, pages 405 - 411
- SHAPIRO, J.I.: 'Cyclin-dependent kinase pathways as targets for cancer treatment' J.CLIN.ONCOL. vol. 24, no. 11, 2006, pages 1770 - 1783, XP009148872
- ROSE-HELLEKANT, T.A. ET AL.: 'Transforming growth factor alpha- and c-myc-induced mammary carcinogenesis in transgenic mice' ONCOGENE vol. 19, 2000, pages 1092 - 1096, XP055098837
- SANDGREN, E.P. ET AL.: 'Inhibition of mammary gland involution is associated with transforming growth factor a but not c-myc- induced tumorigenesis in transgenic mice' CANCER RES. vol. 55, 1995, pages 3915 - 3927, XP055098831
- PARK, E-J. ET AL.: 'Down-regulation of c-src/ EGFR-mediated signaling activation is involved in th honokiol-induced cell cycle arrest and apoptosis in MDA-MB-231 human breast cancer cells' CANCER LETT. vol. 277, 2009, pages 133 - 140, XP026072787
- BOTCHKAREV, V.A. ET AL.: 'p53 Involvement in the control of murine hair follicle regression' AM.J.PATHOL. vol. 158, no. 6, 2001, pages 1913 - 1919, XP002996833
- RANGARAJAN ANNAPOORNI ET AL: "Species- and cell type-specific requirements for cellular transformation.", CANCER CELL AUG 2004, vol. 6, no. 2, August 2004 (2004-08), pages 171-183, ISSN: 1535-6108
- HJELLE B ET AL: "ONCOGENE V-SRC TRANSFORMS AND ESTABLISHES EMBRYONIC RODENT FIBROBLASTS BUT NOT DIPLOID HUMAN FIBROBLASTS", PROCEEDINGS OF THE NATIONAL ACADEMY OF SCIENCES OF THE UNITED STATES OF AMERICA, vol. 85, no. 12, 1988, pages 4355-4359, ISSN: 0027-8424

**Description**

TECHNICAL FIELD

The present invention relates to a method for producing tumor cells based on normal mammary epithelial cells without using TERT, tumor cells produced according to this method, and a method for screening antitumor agents using the tumor cells.

BACKGROUND ART

Breast cancer is characterized as a tumor that occurs in mammary tissue, and 10% of women in Western countries are known to be afflicted with breast cancer at some point in their lives. In addition, approximately 20% of women with breast cancer end up dying due to the cancer. Moreover, although occurring at a lower rate (about 0.1 %), breast cancer is also known to occur in men.

The mechanism of the occurrence of breast cancer is still not figured out systematically. Consequently, the current treatment of breast cancer is forced to rely on the surgical excision of cancerous tissue as a general rule, with chemotherapy and radiotherapy being limited to the roles of secondary treatment. Among the types of breast cancer, a type known as "triple negative" breast cancer, which is negative for expression of estrogen receptor, negative for expression of progesterone receptor and negative for erthythroblastic leukemia viral oncogene homolog 2 (ERBB2) protein, in particular advances rapidly and has a poor prognosis, and therefore an effective treatment method is needed.

In recent years, development of *in vitro* cancer model systems has been proceeding by genetically manipulating normal human cells. Although conventional cell lines established from tumor tissue contained unknown genetic abnormalities and therefore were not suitable for research on tumorigenesis induced by gene alterations, several *in vitro* cancer model systems as described above are useful for directly determining the effects of specific gene alterations on tumorigenesis. For example, Non-Patent Document 1 reports the first successful malignant transformation of mammary epithelial cells by introducing human telomere reverse transcriptase (hTERT) gene, SV40T antigen gene and HRAS$^{V12}$ gene into normal mammary epithelial cells. In addition, Kendall, S.D. et al. succeeded in producing breast cancer cells by introducing hTERT gene, TP53 mutant gene, cyclin D1 gene, Cdk4 mutant gene and HRAS$^{V12}$ gene into normal mammary epithelial cells (Non-Patent Document 2). Moreover, an example of malignant transformation of normal human mammary epithelial cells has been reported in which a different gene combination consisting of hTERT gene, TP53 mutant gene, HRAS$^{V12}$ gene and PIK3CA mutant gene were introduced therein (Non-Patent Document 3).

However, despite the progress being made by these studies, there are currently hardly any reports describing the artificial production of breast cancer cells demonstrating a pathologically high degree of similarity with breast cancer cells isolated from patients. In particular, there have been no reports of successful cases of production of triple negative breast cancer cells.

Although cancer cell lines isolated from cancer patients are currently used in cancer cell research and the development anticancer agents, there are variations in the status of gene mutation between these cell lines since nearly all of such patients derived cancer cell lines have many unspecified genetic damages,. Thus, when such cells are used in research on specific molecules or signal pathways, the experimental results obtained cannot be applied to different cell lines. In addition, since these cells have unspecified genetic damages, there are cases in which they cannot be used in experiments for evaluating the involvement of individual genes in malignant transformation.

US 2006/0222590 A1 discloses a method of producing a tumorigenic mouse cell, the tumorigenicity of which depends on a recombinant gene of interest. The method involves: (a) providing a conditionally tumorigenic mouse cell containing a recombinant oncogene operably linked to an inducible promoter, wherein (i) expression of the recombinant oncogene is necessary and sufficient for the tumorigenicity of the tumorigenic mouse cell, and (ii) the inducible promoter is in the uninduced state; and (b) introducing into the cell a recombinant gene of interest that functionally complements the oncogene. Also disclosed is a method of testing a compound for anti-tumor effects. The method includes producing tumorigenic mouse cells the tumorigenicity of which depends on expression of a recombinant gene of interest, implanting the cells in mice and obtaining tumors from the implanted cells, administering test compounds to the mice, and determining anti-tumor effects, if any, of the compounds.

Non-Patent Document 1: Elenbaas, B. et al., Genes Dev. 2001, 15: 50-65
Non-Patent Document 2: Kendall, S. Disean., Cancer Res. 2005, Nov. 1; 65(21): 9824-9828
Non-Patent Document 3: Zhao, J.J., Proc. Natl. Acad. Sci. USA 2006, 31; 103(44): 16296-16300

DISCLOSURE OF THE INVENTION

With the foregoing in view, there is a need for the development of cancer model cells in which the status of gene mutation is defined, and particularly the development of cancer models cells for triple negative breast cancer.

The inventors of the present invention succeeded in inducing malignant transformation in normal human mammary epithelial cells by genetically manipulating those cells. Since the tumor cells obtained in this manner underwent malignant transformation as a result of genetic manipulation of normal cells, the status of gene mutation is defined. When these tumor cells obtained by the inventors of the present invention were observed in detail, they were revealed to exhibit histological characteristics of human breast cancer cells, and particularly those of triple negative breast cancer cells. The above discovery led to the present invention.

Namely, the present invention relates to that indicated below.

[1] A method for producing tumor cells by carrying out the following treatments on normal human mammary epithelial cells:

(1) elimination of p53 function; and
(2) introduction v-Src gene;

wherein the tumor cells are negative for expression of estrogen receptor, negative for expression of progesterone receptor, and negative for expression of ERBB2.

[2] The method described in [1], wherein introduction of a cyclin-dependent kinase gene is further carried out.

[3] The method described in [2], wherein the cyclin-dependent kinase gene is Cdk4 gene.

[4] Tumor cells produced according to the method described in any one of [1] to [3].

[5] A method for screening anti-tumor agents, this method comprising:

(a) contacting the tumor cells described in [4] with a candidate substance; and
(b) detecting growth inhibitory effects on the tumor cells.

[6] A cancer-bearing non-human animal model transplanted with the tumor cells described in [4].

[7] The animal model described in [6], wherein the animal model is selected from mice, rats, pigs, dogs, monkeys, hamsters and rabbits.

[8] A method for screening anti-tumor agents, this method comprising:

(a) contacting the cancer-bearing animal model described in [6] or [7] with a candidate substance; and
(b) detecting growth inhibitory effects on tumor cells.

Since tumor cells produced according to the method of the present invention undergo malignant transformation by genetically manipulating normal cells, the gene mutation status thereof is defined. Thus, according to the present invention, a model system can be provided that is extremely useful for evaluating the involvement of individual genes in the development of breast cancer. In addition, since tumor cells produced according to the method of the present invention demonstrate the phenotype of triple negative breast cancer, they have the potential to be extremely useful in the development of treatment methods and therapeutic drugs for triple negative breast cancer, which has conventionally been difficult to treat.

BRIEF DESCRIPTION OF THE DRAWINGS

FIG. 1 is a drawing indicating the anatomical origin of normal human mammary epithelial cells in an embodiment of the present invention;

FIG. 2 is a drawing indicating a method for producing tumor cells from normal mammary epithelial cells according to an embodiment of the present invention;

FIG. 3 is a drawing indicating the results of confirming expression and function of genes introduced into tumor cells of the present invention by Western blotting, with FIG. 3A indicating the results for expression of Src gene, FIG. 3B indicating the results for expression of p53 gene, and FIG. 3C indicating the results for phosphorylation of tyrosine by introduced Src gene;

FIG. 4A is a graph indicating the tumor tissue formation ability of tumor cells of the present invention, while FIG. 4B depicts photographs showing tumor tissue formed by transplanting tumor cells of the present invention into nude mice;

FIG. 5 is a drawing indicating the pathological characteristics of tumor tissue formed by transplanting tumor cells of the present invention (HME/53/v-Src cells) into nude mice;

FIG. 6 is a drawing indicating the pathological characteristics of tumor tissue formed by transplanting tumor cells of the present invention (HME/53/Cdk4/v-Src cells) into nude mice;

FIG. 7 indicates the results of having formed a tumor mass by transplanting tumor cells of the present invention produced without introducing TERT gene into nude mice; and

FIG. 8 is a drawing indicating the pathological characteristics of tumor tissue formed by transplanting tumor cells of the present invention (HME/53/EGFR$^{T790.L858R}$/c-Myc cells) into nude mice.

## BEST MODE FOR CARRYING OUT THE INVENTION

The following provides a detailed explanation of the present invention. The following embodiments are intended to be exemplary for the purpose of explaining the present invention, and are not intended to limit the present invention thereto. The present invention can be carried out in various forms provided they do not deviate from the claims.

1. Method for Producing Tumor Cells

In a first aspect, the present invention provides a method for producing tumor cells by carrying out the following treatments on normal human mammary epithelial cells. Note that there are no particular limitations on the order in which treatment is carried out:

(1) elimination of p53 function; and
(2) introduction v-Src gene.

In the above-mentioned aspect, the method of the present invention may also include: forcibly expressing a cyclin-dependent kinase gene as a third treatment (3) in addition to the treatments described above.

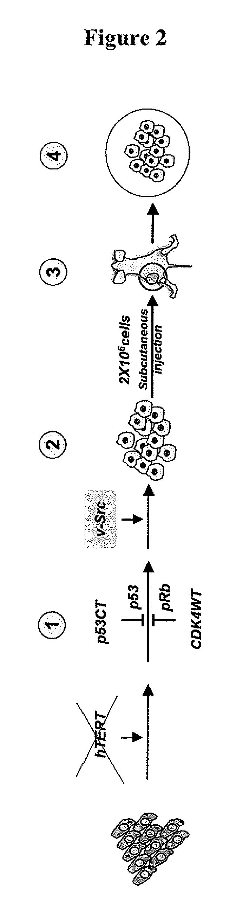

The method of the present invention is characterized by inducing malignant transformation of normal mammary epithelial cells without introducing TERT gene (see FIG. 2). There have previously been no reports of examples of successfully producing tumor cells without introducing TERT gene in a method for producing tumor cells from normal cells by expression of an exogenous gene.

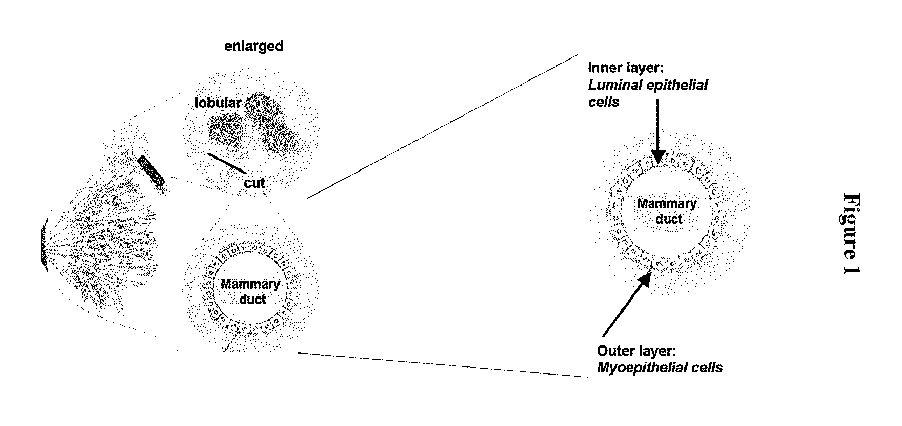

In the present invention, there are no particular limitations on the "normal human mammary epithelial cells" provided they are normal cells derived from human mammary epithelium. Mammary glands are known to be developed in mammals such as humans, monkeys and mice. In the present invention, "normal human mammary epithelial cells" are preferably normal cells originating in human mammary gland epithelium or mammary duct epithelium. The normal cells are still more preferably normal cells originating in myoepithelium that forms the outer layer of the mammary duct (myoepithelial cells) (see FIG. 1). "Normal cells" refer to cells in a healthy state, or in other words, are in a state free of detectable diseases or abnormalities, while "normal human mammary epithelial cells" refers to human mammary epithelial cells in a state free of detectable diseases or abnormalities in the manner of human mammary epithelial cells from healthy subject, for example. In the following descriptions, "human mammary epithelial cells" are referred to as "human myoepithelial cells (HME cells)".

HME cells harvested from human subjects may be used, or commercially available cells may be used (such as cells available from Lonza Inc. (Walkersville, MD, USA)).

"Elimination of p53 function" refers to p53 protein not having its inherent biological function.

p53 protein is encoded by TP53 gene, and TP53 gene is a gene that encodes p53 protein involved in activation of DNA repair protein, control of the cell cycle and induction of apoptosis, and functional abnormalities thereof are known to be related to the onset of various cancers (Lane, D.P. (1992) Nature 358: 15-16).

Various methods can be applied to eliminate p53 function, and although there are no particular limitations thereon provided it eliminates p53 function, examples of such methods include addition of p53 protein neutralizing antibody, cellular introduction of a gene that encodes that antibody, knockout of a gene that encodes p53 protein (to be referred to as "TP53 gene"), knockdown of TP53 gene by RNA interference (see Sato, M. et al. (Cancer Res. 66, (2006) 2116-2128)), and forced expression of dominant negative TP53 gene. Introduction of dominant negative TP53 gene (also referred to as "p53CT gene") is preferably used in the present invention to eliminate p53 function. The nucleotide sequence of dominant negative TP53 gene can be ascertained by referring to the reference of Shaulian, E. et al. (Mol. Cell. Biol., Dec. 1992; 12: 5581). The TP53 gene or p53 protein used when eliminating the function of p53 protein is preferably a gene or protein of mammalian origin, more preferably a gene or protein of primate origin, and even more preferably a gene or protein of human origin.

Furthermore, the function of other apoptosis-inducing proteins such as caspase-3, -8 to -10 or -12 may be eliminated instead of eliminating the function of p53. In this case as well, protein function can be eliminated using a similar method as that employed when eliminating the function of p53.

"v-Src gene" was discovered as a cancer-related gene originating in Rous sarcoma virus, which is a type of retrovirus, and the sequence thereof is described in Mayer, B.J. et al. (J. Virol. 1986 Dec.; 60(3): 858-67).

"Cyclin-dependent kinase (Cdk) gene" is a gene that encodes protein involved in progression of the cell cycle, and the family of which it is a member is composed of Cdk1 to Cdk13. Although the Cdk gene introduced into normal human mammary epithelial cells in the present invention may be a gene that encodes any of Cdk1 to Cdk13, a gene that encodes Cdk4 (Cdk4 gene) can be used preferably. Cdk4 is the binding partner of cyclin D1, and mutations of Cdk4 gene have been detected in various types of tumors (Zuo, L. et al. (1996), Nature Genet. 12, 97-99). The nucleotide sequence registered in the NCBI database is used for the nucleotide sequence of Cdk gene. In the present invention, Cdk gene is preferably a mammalian Cdk gene, more preferably a primate Cdk gene, and even more preferably a human Cdk gene.

"EGFR gene" is a gene that encodes epithelial growth factor receptor protein, and elevated expression of EGFR gene is observed in numerous cancers, including lung cancer, breast cancer, colorectal cancer, stomach cancer and brain cancer (Sharma, S.V et al., Nature Rev. Cancer 2007; 7(3): 169-81).

"EGFR mutant gene" is an EGFR gene that has mutated from the wild type, and an example of which is a gene that encodes EGFR$^{T790M.L858R}$ mutant protein (SEQ ID NO: 10) in which a threonine residue at position 790 has been substituted to methionine and a leucine residue at position 858 has been substituted to arginine. The nucleotide sequence of EGFR mutant gene can be produced according to the methods described in McCoy, M.S. et al. (Mol. Cell. Biol., (1984), 4, 1577-1582), Samuels, Y. et al. (Science (2004), 304, 554) or Scott, K.D. et al. (Cancer Res. (2007), 67, 5622-5627) based on the wild type nucleotide sequence registered in the NCBI database. EGFR gene is preferably mammalian EGFR gene, more preferably primate EGFR gene and
even more preferably human EGFR gene.

"c-Myc gene" is a gene that encodes DNA binding factor protein involved in regulating the expression of various genes and DNA replication, or in other words, a transcription factor gene, abnormalities in the expression of c-Myc gene are suggested to be related to various cancers (Dominguez-Sola, D. et al. (2007) Nature 448, 445-451). The nucleotide sequence registered in the NCBI database is used for nucleotide sequence of c-Myc gene. c-Myc gene is preferably mammalian c-Myc gene, more preferably primate c-Myc gene and even more preferably human c-Myc gene.

The nucleotide sequences and peptide sequences of the various genes and proteins used in the examples to be subsequently described are indicated in the following Table 1, although not limited thereto.

Table 1

| Gene Name | NCBI Accession No. | Species | Sequence No. (gene) | Sequence No. (protein) |
|---|---|---|---|---|
| TP53 gene | NM_000546.4 | Homo sapiens | -- | -- |
| Dominant negative TP53 gene | -- | Homo sapiens | 1 | 2 |
| v-Src gene | -- | Rous sarcoma virus | 3 | 4 |
| Cdk4 gene | NM_000075.2 | Homo sapiens | 5 | 6 |
| EGFR gene | NM_005228.3 | Homo sapiens | -- | -- |
| EGFR mutant gene | -- | Homo sapiens | 9 | 10 |
| c-Myc gene | V00568.1 | Homo sapiens | -- | -- |

Gene Introduction Method

In the case of introducing a gene into HME cells in the present invention, the gene is inserted into a suitable expression cassette in the form of an expression vector, and the HME cells are transformed with the expression vector. A suitable expression cassette at least contains the following constituents (i) to (iii):

    (i) promoter capable of transcribing in HME cells;
    (ii) gene ligated in-frame to the promoter; and
    (iii) sequence encoding transcription termination and polyadenylation signal of RNA molecule.

Examples of promoters capable of transcribing in HME cells include, but are not limited to, CMV, CAG, LTR, EF-1$\alpha$ and SV40 promoters.

The above-mentioned expression vector may also have a selection marker expression cassette for selecting transformed HME cells in addition to the above-mentioned expression cassette. Examples of selection markers include, but are not limited to, positive selection markers such as neomycin resistance gene or hygromycin B phosphotransferase gene, expression reporters such as LacZ, green fluorescent protein (GFP) or luciferase gene, and negative selection markers such as herpes simplex virus thymidine kinase gene (HSV-TK) or diphtheria toxin A fragment (DTA).

Transformed HME cells can be easily selected with the above-mentioned markers. For example, in the case of cells introduced with a marker in the form of neomycin resistance gene, primary selection can be carried out by culturing in medium containing G418. In addition, in the case of containing a targeting vector in the form of a fluorescent protein gene such as GFP, in addition to selecting on the basis of drug resistance, sorting of cells expressing fluorescent protein may be carried out using a fluorescence-activated cell sorter (FACS).

Examples of expression vectors able to be used to introduce a gene into HME cells include expression vectors capable of introducing genes into cells, and commercially available expression vectors may also be used. Examples of such commercially available expression vectors include pEGFP-C1™ (Clontech), pCMV-HA™ (Clontech), pMSCVpuro™ (Clontech), pEF-DEST51™ (Invitrogen), pCEP4™ (Invitrogen) and ViraPower II Lentiviral Gateway System™ (Invitrogen). The expression vector can be introduced into HME cells by a known gene introduction method such as electroporation, microinjection, calcium phosphate method, lipofection or viral infection. Reference can be made to "Sambrook & Russell, Molecular Cloning: A Laboratory Manual, Vol. 3, Cold Spring Harbor Laboratory Press 2001 ", for example, for details on gene introduction methods.

2. Tumor Cells

In the present invention, "tumor cells" refer to cells that autonomously undergo overgrowth in vivo, and may not only be tumor cells produced in the above-mentioned "method for producing tumor cells", but may also be tumor cells isolated from a "cancer-bearing animal model" to be subsequently described or cells obtained by culturing, in vitro, tumor cells isolated from this cancer-bearing animal model.

Examples of tumor cells include cells contained in breast cancer tumors. In one embodiment of the present invention, a preferable form of the tumor cells is breast cancer cells. The tumor cells are triple negative breast cancer cells negative for all of the markers consisting of estrogen receptor (ER), progesterone receptor (PgR) and ERBB2 (also known as "HER2") protein.

In the case of confirming malignant transformation of cells, the test cells are subcutaneously injected into a suitable non-human animal model and confirming malignant transformation by observing the formation of a tumor mass.

A mammal other than a human is preferable for the animal model, while an immunosuppressed mammal is particularly preferable. Examples of immunosuppressed mammals include, but are not limited to, nude rats and nude mice.

Another example of a method for confirming malignant transformation of cells consists of culturing test cells on soft agar and observing their colony formation (soft agar colony formation assay method). As a specific example thereof, HME cells introduced with TP53 mutant gene and v-Src gene are disseminated in soft agar medium after adjusting to a constant cell concentration followed by observation of cell growth rate. Reference can be made to Tanaka, S. et al., Proc. Natl. Acad. Sci. USA, 94: 2356-2361, 1997 for details on the soft agar colony formation assay method.

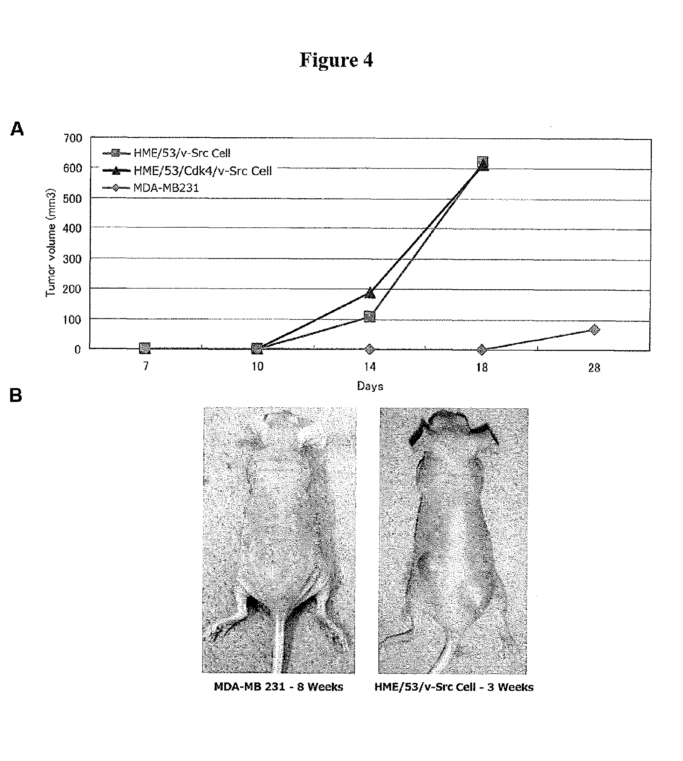

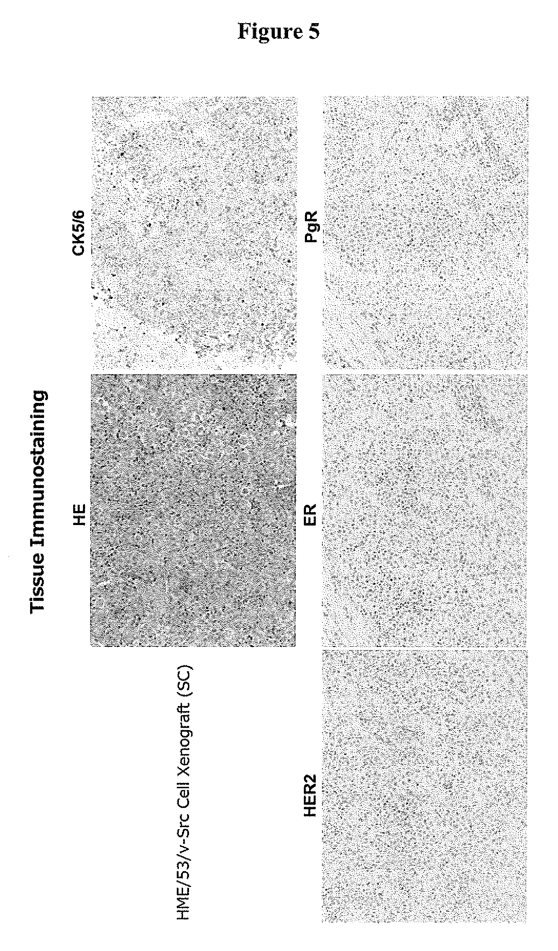

Tumor cells obtained in the present invention (to be referred to as "tumor cells of the present invention") are characterized by having extremely high proliferation ability. As shown in FIG. 4A, tumor cells of the present invention have a remarkably high proliferation ability in comparison with human breast cancer cell line MDA-MB231 cells (ATCC HTB-26). In addition, in an embodiment thereof, tumor tissue obtained by transplanting the tumor cells of the present invention into an animal model were ER(-), PgR(-) and ERBB2(-), were ER(-), PgR(-) and HER2 (-) in the case of using human mammary epithelial cells, and exhibited the morphology of triple negative breast cancer cells (FIGS. 5, 6 and 8).

In tumor cells observed in human triple negative breast cancer, since the hormone receptors of ER and PgR are not expressed, and since the receptor tyrosine kinase of HER2 is also not expressed, hormone therapy or chemotherapy cannot be expected to be therapeutically effective, and triple negative breast cancer is considered to be the type of breast cancer that is most difficult to treat for this reason.

The tumor cells of the present invention have both a high tumorigenesis rate and high proliferation ability. In the case of forming a tumor by transplanting the tumor cells of the present invention into an animal model in particular, tumors are formed in the animal model that are pathologically extremely similar to tumor tissue spontaneously arising from the mammary gland (duct) epithelial cells in the animal species. For example, in the case of transplanting human mammary gland epithelial cells, tumors are formed in an animal model that are pathologically extremely similar to cancer tissue isolated from human cancer patients, and particularly triple negative breast cancer tissue. Thus, the tumor cells of the present invention have the potential to be extremely useful in the development of treatment methods or therapeutic drugs for triple negative breast cancer.

3. Cancer-Bearing Non-Human Animal Model

In the present invention, a "cancer-bearing non-human animal model" refers to an animal in which a tumor mass has been formed by transplanting the above-mentioned tumor cells into an animal model other than a human. A preferable example of a non-human animal model is a mammal other than a human, examples of which include, but are not limited to, mice, rats, pigs, dogs, monkeys, hamsters and rabbits. Among these animal models, mammals are preferable, and primates or rodents are more preferable. Among these, immunosuppressed mammals are particularly preferable, while immunosuppressed primates or rodents are even more preferable. Although immunosuppressed mammals can be produced by administering an immunosuppressant such as cyclosporin to an ordinary mammal, mammals in which immunity has been congenitally suppressed based on genetic background are preferable. Examples of mammals in which immunity has been congenitally suppressed include, but are not limited to, nude rats and nude mice.

There are no particular limitations on the method used to transplant the tumor cells into the animal model. A method conventionally used corresponding to the animal model to be transplanted with the tumor cells may be suitably selected. Reference can be made to Genetic Induction of Tumorigenesis in Swine, Oncogene 26, 1038-1045 (September 11, 2006), for example, for examples of transplanted animal models other than mice. From the viewpoint of ease of re-excision of the transplanted tumor cells, transplantation is preferably carried out by subcutaneous injection or intraperitoneal injection, while local transplantation is preferable from an anatomical viewpoint.

4. Method for Screening Antitumor Agents

The present invention provides a method for screening anti-tumor agents.

In a first aspect thereof, as the screening method of the present invention, a method for screening anti-tumor agents is provided that includes:

(a) contacting the tumor cells of the present invention with a candidate substance; and
(b) detecting growth inhibitory effects on the tumor cells.

In addition, in a second aspect thereof, as the screening method of the present invention, a method for screening anti-tumor agents is provided that includes:

(a) contacting a cancer-bearing non-human animal model transplanted with the tumor cells of the present invention with a candidate substance; and
(b) detecting growth inhibitory effects on the tumor cells.

In the above-mentioned descriptions, "contacting the tumor cells with a candidate substance" or "contacting a cancer-bearing non-human animal model transplanted with tumor cells with a candidate substance" refers to the candidate substance approaching the tumor cells to a degree that it interacts with molecules on the surface thereof or binds with those molecules, or adjusting conditions at which the candidate substance is taken into the tumor cells. In the case the tumor cells are tumor cells such as cultured cells, a candidate substance can be allowed to contact the cells by adding the candidate substance to a culture medium contacted by the cells at a fixed concentration or more. On the other hand, in the case the tumor cells have been transplanted into anon-human animal body, namely in the case of a cancer-bearing non-human animal model transplanted with the tumor cells, a candidate substance can be allowed to contact the tumor cells by administering the candidate substance to the animal at a fixed dosage. In this case, although there are no particular limitations on the administration route provided it is a route that is commonly employed to administer a candidate substance, and specific examples include oral, sublingual, pernasal, intrapulmonary, alimentary, percutaneous, instillation, intravenous injection, subcutaneous injection, intramuscular injection, intraperitoneal injection, local injection and surgical transplant, with oral administration, intraperitoneal injection and intravenous injection being preferable.

Examples of candidate substances include compounds drugs for which antitumor effects have already been confirmed as well as compounds, polypeptides, nucleic acids, antibodies and low molecular-compounds having the potential to exhibit antitumor effect. Specific examples of such candidate substances include, but are not limited to, metabolic antagonists (such as 5-fluorouracil (5-FU)), folate metabolism antagonists (including dihydropteroic acid synthase inhibitors such as sulfadiazine or sulfamethoxazole and dihydrofolate reductase inhibitors (DHFR inhibitors) such as methotrexate, trimethoprim or pyrimethamine), pyrimidine metabolism inhibitors (including thymidylate synthase inhibitors such as 5-FU or flucytosine (5-FC)), purine metabolism inhibitors (including IMPDH inhibitors such as 6-mercaptopurine and the prodrugs such as azathioprine), adenosine deaminase (ADA) inhibitors (such as pentostatin), ribonucleotide reductase inhibitors (such as the ribonucleotide reductase inhibitor, hydroxyurea), nucleotide analogs (including purine analogs such as thioguanine, fludarabine phosphate or cladribine as well as pyrimidine analogs such as cytarabine or gemcitabine), L-asparaginase, alkylating agents (including nitrogen mustard agents such as cyclophosphamid, melphalan or thiotepa, platinating agent preparations such as cisplatin, carboplatin or oxaliplatin, and nitrosourea drugs such as dacarbazine, procarbazine or ranimustine), antitumor antibiotics (such as sarcomycin, mitomycin C, doxorubicin, epirubicin, daunorubicin or bleomycin), topoisomerase inhibitors (such as irinotecan, nogitecan, doxorubicin, etoposide, levofloxacin or ciprofloxacin), microtubule polymerization inhibitors (such as vinblastine, vincristine or vindesine), colchicine, microtubule depolymerization inhibitors (such as paclitaxel or docetaxel), molecular-targeted agents (such as trastuzumab, rituximab, imatinib, gefitinib, bortezomib or erlotinib), steroids such as dexamethasone, finasteride, aromatase inhibitors, tamoxifen and combinations thereof.

Growth inhibitory effects on tumor cells can be confirmed by producing two systems of cultures of the same cells disseminated in culture dishes at the same cell density and cultured under the same conditions or two cancer-bearing non-human animal models of the same strain transplanted with the same number of cells at the same cell density, contacting the above-mentioned candidate substance with cells of one of the systems (sample), not contacting the candidate substance with cells of the other system (control), observing proliferation of the tumor cells of both systems, and measuring and comparing the number of tumor cells contained in each of the two systems after a fixed period of time has elapsed.

In the case of screening the tumor cells of the present invention in the form of tumor cells such as cultured cells, tumor growth effects can be confirmed by contacting a candidate substance with the above-mentioned sample cells, measuring the number of sample cells and the number of control cells with a cell counter and the like, and comparing the numbers of each of the cells.

In the case of screening the tumor cells of the present in the form of transplanting into anon-human animal model, namely in the case of screening in the form of anon-human animal model in which the tumor cells have been transplanted, tumor growth effects can be confirmed by administering a candidate substance to the animal model of a sample system, extracting tumor tissue from animals of the sample system and control system, and measuring and comparing the number of cells contained in the tumor tissue from the sample system and control system. Alternatively, tumor growth effects can also be confirmed by extracting the above-mentioned tumor tissue, and comparing the volume of the tumor tissue between a sample system and a control system. Tumor volume can be determined with the following equation.

$$\text{Tumor volume} = ab2/2 \text{ (a: horizontal width, b: length)}$$

Alternatively, tumor growth effects can also be confirmed by administering a candidate substance to animals of a sample system and a control system, and comparing rate at which a measurable tumor mass is formed at the transplanted location of the tumor cells between the sample system and the control system.

The non-human animal model is the same as the animal model described in section 3 entitled "Cancer-Bearing Non-Human Animal Model". Although there are no limitations on the method used to transplant tumor cells into the animal model, subcutaneous injection or intraperitoneal injection is preferable in consideration of the ease of re-excision of the transplanted tumor cells.

In the case the rate of increase of tumor cells of the sample system is less than the rate of increase of tumor cells of the control system, the candidate substance used can be judged to have antitumor effects. Alternatively, in the case multiple sets of data on the rate of increase under fixed conditions are already available for the tumor cells of the present invention, an assessment may also be made by statistically processing that data, and comparing standard values derived from the resulting average values, standard deviations and the like.

Although average values or standard deviations and the like of tumor growth rates can be obtained by various statistical methods, more specifically, these values can be determined by two-way ANOVA using statistical processing software such as IBM SPSS Statistics 18 (SSPS) using the initial number of cells and cell density at the time of cell dissemination as parameters in the case of cultured cells, or using body weight of an animal model at the time of transplant and the number of transplanted tumor cells in the case of transplanted cells. Analysis accuracy can be further improved by using the growth rate of tumor cells obtained by carrying out the method of the present invention as new data and adding to the population used for statistical analysis to increase statistical parameters.

Since the tumor cells of the present invention demonstrate characteristics that are pathologically extremely similar to breast cancer cells isolated from actual patients, antitumor agents that have been confirmed to demonstrate growth inhibitory effects on tumor cells as determined with the screening method of the present invention are expected to demonstrate antitumor effects in cases of actually using in the treatment of cancer patients, and particularly breast cancer patients (such as triple negative breast cancer patients).

Although the following provides a detailed explanation of the present invention using an example thereof, the present invention is not limited to the aspects described in the example.

[Example]

In the present example, tumor cells were produced by introducing the gene combinations shown in Table 1 to normal HME cells.

In the following descriptions, each of the produced tumor cells is indicated using the nomenclature shown in the following table corresponding to the type of gene introduced into the HME cells.

Table 2

| Genes Introduced into HME Cells | Tumor Cell Nomenclature |
|---|---|
| Dominant negative TP53 (p53CT) gene | HME/53 cells |
| Dominant negative TP53 gene and v-Src gene | HME/53/v-Src cells |
| Dominant negative TP53 gene and Cdk4 gene | HME/53/Cdk4 cells |
| Dominant negative TP53 gene, Cdk4 gene and v-Src gene | HME/53/Cdk4/v-Src cells |
| Dominant negative TP53 gene and c-Myc gene | HME/53/c-Myc cells |
| Dominant negative TP53 gene and EGFR$^{T790.L858R}$ gene | HME/53/EGFR$^{T790.L858R}$ cells |
| Dominant negative TP53 gene, EGFR$^{T790.L858R}$ gene and c-Myc gene | HME/53/EGFR$^{T790.L858R}$/c-Myc cells |

The following provides a description of the experimental procedure carried out in the present example.

Cell Culture

Normal human mammary epithelial cells derived from a 30-year-old Caucasian woman were purchased from Lonza Inc. (Walkersville, MD, USA)) for use as HME cells. The cells were cultured in a collagen-coated dish in serum-free MEGM medium (MEGM Bullet Kit, Lonza Inc.) containing various growth factors provided by Lonza Inc. The cells were cultured in a humid incubator maintained at 37°C in a low oxygen environment (3% $O_2$ and 5% $CO_2$), and were used in the present example as HME cells.

Production of Retrovirus Vector and Introduction of Retrovirus vector into HME Cells

Vectors for retrovirus expression were produced by incorporating the v-Src, Cdk4, dominant negative TP53, EGFR$^{T790.L858R}$ and c-Myc genes shown in Table 1 into each of the vectors shown in the following Table.

Table 3

| Insert Gene | Expression Vector |
|---|---|
| v-Src | pCX4pur vector (GenBank Accession No: AB086386) |
| Cdk4 | pCX4bsr vector (GenBank Accession No: AB086384) |
| Dominant negative TP53 | pCX4.1hisD vector (GenBank Accession No: AB086389) |
| EGFR$^{T790.L858R}$ | pCX4pur vector (GenBank Accession No: AB086386) |
| c-Myc | pCX4bleo vector (GenBank Accession No: AB086388) |

Virus vectors were produced by introducing the above-mentioned retrovirus expression vectors along with pGP and pE-eco plasmids purchased from Takara Bio Inc. (Shiga, Japan) into 293T cells. Subsequently, the above-mentioned virus vectors were infected into HME cells that expressed Ecotropic receptor (Eco VR).

The cells infected with the virus vectors were subjected to selection by culturing for 2 weeks in the presence of puromycin and bleomycin. The cultured cells were selected from groups of cultured cells exhibiting polyclonal proliferation when carrying out selection using either of the drugs.

Furthermore, HME cells expressing ecotropic receptor (Eco VR) were produced according to the procedure indicated below.

cDNA containing the entire encoding region of mouse ecotropic retrovirus receptor (Slc7al, NM_007513) was cloned by RT-PCR using the primers indicated below.

FW primer: 5'-GATCCTCCCCAGTGAGAAGT-3' (SEQ ID NO: 7)
RV primer: 5'-CTCACTAGCCATCTGGAGTG-3' (SEQ ID NO: 8)

The amplification product of the above-mentioned RT-PCR was incorporated in pCx4hyg vector (GenBank Accession No: AB086387). A virus vector was then produced by introducing this vector along with plasmids pGP and pE-Ampho purchased from Takara Bio Inc. (Shiga, Japan) into 293T cells, and the virus vector was infected into HME cells followed by carrying out selection by culturing for 2 weeks in the presence of hygromycin.

Immunoblotting

Protein assay, SDS-PAGE and immunoblotting were carried out in accordance with the description of a previous reference (Akagi, T. et al. (2002), Mol. Cell. Biol., 22, 7015-7023). The signals of proteins exhibiting a positive immune response were visualized by chemiluminescence using the SuperSignal WestFemto reagent (Pierce Inc.). Anti-Src antibody, anti-phospho-Src (Tyr416) antibody and anti-p53 antibody were purchased from Cell Signaling Technology Inc., while anti-phospho-tyrosine antibody (4G10) was purchased from Millipore Corp.

Xenograft Growth Experiment

A xenograft growth experiment was carried out using mice. More specifically, cell suspensions (single-cell suspensions) containing $1 \times 10^6$ HME cells expressing the gene combinations shown in Table 1 were suspended in 50% Matrigel™ (BD Biosciences Inc., San Jose, CA, USA) and subcutaneously injected into the flanks of 6-week- or 7-week-old female athymic nude mice (BALB/c nu/nu, Japan SLC Inc., Hamamatsu, Japan) or NOD-SCID mice. Following transplantation, the formation of a tumor mass was confirmed visually. During this experiment, formation of a tumor mass was judged to be negative when a volume of a tumor mass does not reach to a degree that it can be pinched between the fingers at 12 weeks after transplantation. Then, the dimensions of the tumors were measured after transplantation using a caliper, and tumor volume was calculated based on the following equation to estimate tumorigenicity.

$$\text{Tumor volume} = \text{ab}^2/2 \text{ (a: horizontal width, b: length)}$$

In addition, the rates at which a measurable tumor mass is formed at the transplanted location of HME cells transduced with each of the cancer-related genes were calculated as a tumorigenesis rate and used to estimate tumorigenicity..

Histological Analysis and Immunohistochemical Staining

Xenografts transplanted in the above-mentioned xenograft growth experiment were removed on day 20 after transplantation. The xenografts were fixed in formalin and embedded in paraffin followed by slicing into sections and subjecting to hematoxylin and eosin (H&E) staining in accordance with the normal protocol. Immunohistochemical staining was then carried out using the following antibodies in accordance with a previous reference (Sasai, K. et al. (2008), Am. J. Surg. Pathol. 32, 1220-1227): cytokeratin 5/6 (CK5/6, Dako Corp., M7237), estrogen receptor (ER, Dako Corp., M7040), progesterone receptor (PgR, Dako Corp., M3569) and HER2 (Dako Corp., K5204).
More specifically, immunohistochemical staining was carried out according to the following procedure. Tissue sections having a thickness of 4 $\mu$m were deparaffinized with xylene and then dehydrated with ethanol. Antigen was retrieved by heating for 2 minutes in 10 mM citrate buffer (pH 6.0) in an autoclave. Endogenous peroxidase was deactivated by rehydrating the tissue sections with phosphate-buffered physiological saline containing 0.01 % Tween 20 (PBST), and incubating with 0.3% hydrogen peroxide. After incubating the tissue sections overnight at 4°C with primary antibody at a suitably diluted concentration and washing with PBST, the tissue sections were incubated for 30 minutes at room temperature in Envision Dual Link Solution (Dako Corp., Glostrup, Denmark). Next, the sections were treated with diaminobenzene (Dako Corp.) to visualize antigen-antibody reaction sites and subjected to nuclear staining by treating with hematoxylin for 90 seconds. Slides of the tissue sections were observed by sealing the tissue sections with a cover glass after mounting with Entellan Neu Reagent (Merck & Co., Whitehouse Station, NJ, USA).

Experiment Results

[1] Confirmation of Expression of Genes Introduced into HME Cells

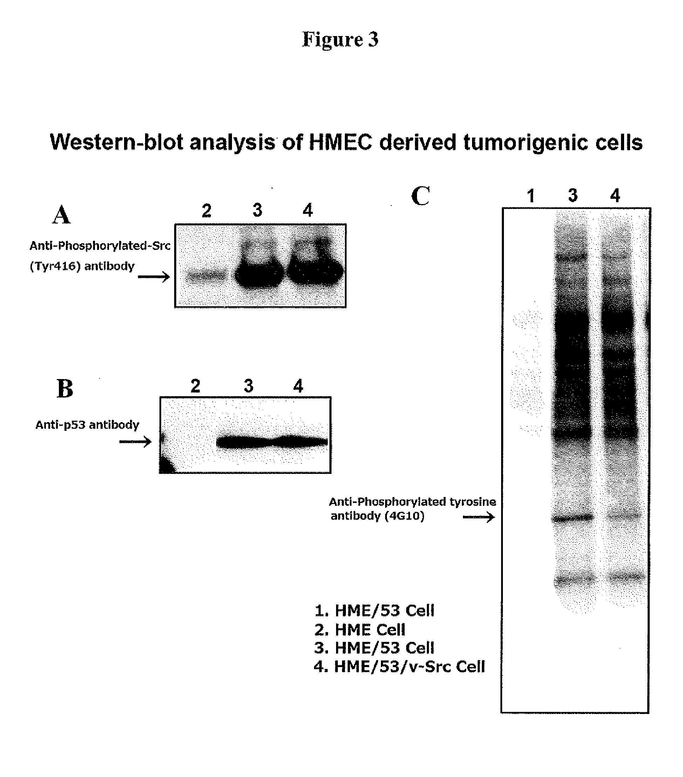

Expression of genes introduced into the HME cells was confirmed by immunoblotting. Expression of dominant negative TP53 gene and v-Src gene introduced into HME/453/v-Src cells and HME/53/v-Src cells was confirmed by antigen-antibody reaction using anti-phospho-Src antibody and anti-p53 antibody (respectively shown in FIGS. 3A and 3B). In addition, increased phosphorylation of tyrosine due to the function of v-Src gene was confirmed with anti-phospho-tyrosine antibody, thereby confirming that the target genes had been introduced and expressed (FIG. 3C).

[2] Confirmation of Tumorigenicity of Produced Tumor Cells

The produced tumor cells were subcutaneously transplanted into nude mice to confirm tumorigenicity of the transplanted tumor graft (FIGS. 4, 7 and 8).

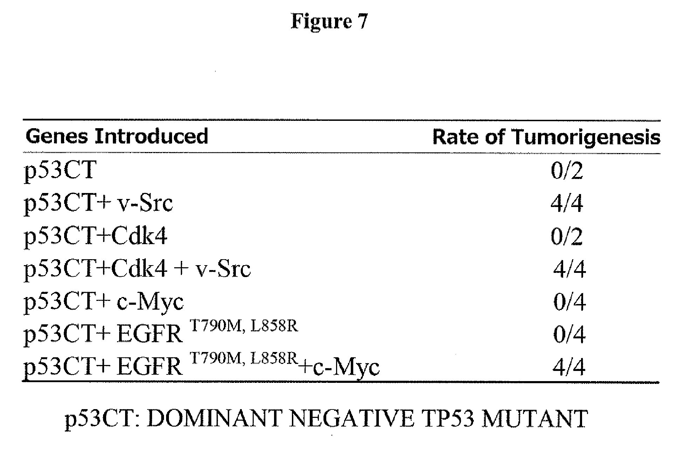

As shown in FIG. 7, formation of a tumor mass was not confirmed in those cells obtained by introducing dominant negative TP53 gene into HMEC cells (HME/53 cells) or in those cells obtained by introducing dominant negative TP53 gene and Cdk4 gene into HMEC cells (HME/53/Cdk4 cells).

As shown in FIGS. 4 and 7, the formation of a tumor mass of a size able to be pinched between the fingers was confirmed in those cells obtained by introducing dominant negative TP53 gene and v-Src gene into HMEC cells (HME/53/Cdk4/v-Src cells and HME/53/v-Src cells), and these cells demonstrated a tumorigenesis rate of 100%. In addition, as shown in FIGS. 4A and 4B, HME/53/Cdk4/v-Src cells and HME/53/v-Src cells clearly demonstrated a high level of proliferation ability since larger tumors were formed in a short period of time as compared with the case of conventionally used human breast cancer cell line MDA-MB231 cells.

As shown in FIG. 7, a tumor mass was not formed in the case of HME/53/EGFR$^{T790.L858R}$ cells and HME/53/c-Myc cells obtained by respectively and independently introducing EGFR$^{T790.L858R}$ gene or c-Myc gene into HMEC cells transduced with dominant negative TP53 gene (HME/53 cells).

On the other hand, formation of a tumor mass was confirmed in the case of HME/53/EGFR$^{T790.L858R}$/c-Myc cells obtained by introducing the combination of dominant negative TP53 gene, EGFR$^{T790.L858R}$ gene and c-Myc gene, and these cells demonstrated a tumorigenesis rate of 100%.

[3] Confirmation of Phenotype of Produced Tumor Cells

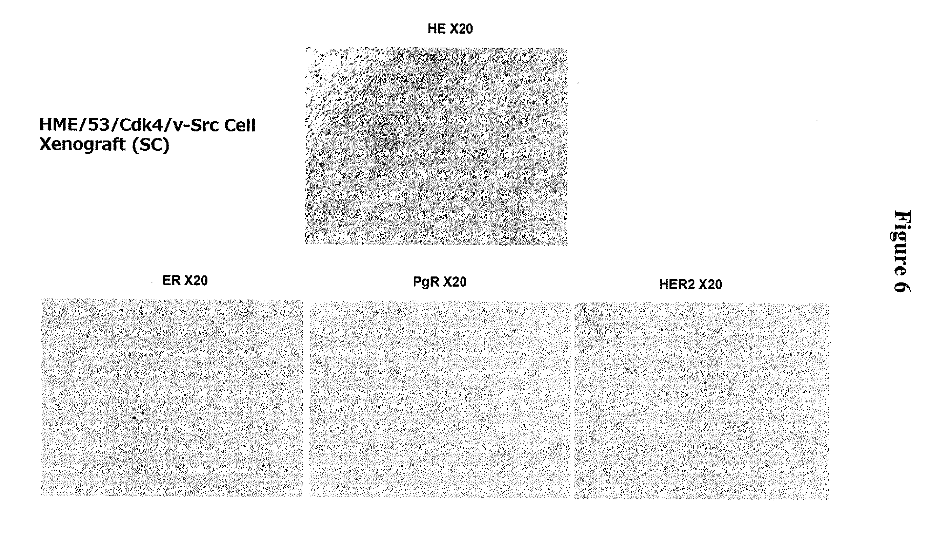

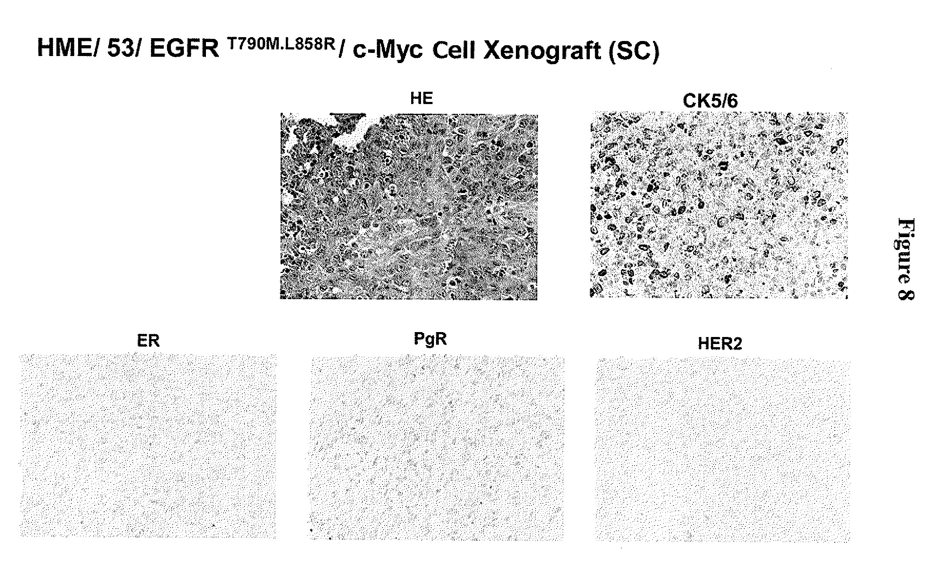

In order to confirm the phenotype of tumor tissue obtained from the xenograft growth experiment, the tumor tissue was immunostained with various cancer markers and observed. When a tissue section of a tumor formed by subcutaneous transplantation of HME/53/v-Src cells was stained with markers used for diagnosis in the clinical setting, the tissue was found to be ER(-), as shown in FIG. 5 PgR(-) and HER2(-) and positive for the Basal type breast cancer marker CK5/6 (CK5/6(+)), thereby demonstrating that HME/53/v-Src cells form tumors that are pathologically extremely similar to triple negative and Basal type breast cancer, which constitute the types of breast cancer that are the most difficult to treat. In addition, as shown in FIG. 6, tissue sections of tumors formed by subcutaneous transplantation of HME/53/Cdk4/v-Src cells were also ER(-), PgR(-) and HER2(-), thereby indicating that these cells similarly form tumors that are pathologically extremely similar to triple negative breast cancer. In addition, as shown in FIG. 8, tissue sections of tumors formed by subcutaneous transplantation of HME/53EGFR$^{T790.L858R}$/c-Myc cells were also ER(-), PgR(-) and HER2(-) and CK5/6(+) for the Basal phenotype breast cancer marker $_{CK5/6}$, HME/53/EGFR$^{T790.L858R}$/c-Myc cells were demonstrated to form tumors that are pathologically extremely similar to triple negative and Basal type breast cancer, which constitute the types of breast cancer that are the most difficult to treat.

As indicated by the above-mentioned results, by introducing a combination of TP53 mutant gene and v-Src gene, a combination of TP53 mutant gene, v-Src gene and Cdk4 gene, or a combination of TP53 mutant gene, EGFR2 mutant gene and c-Myc gene into normal mammary epithelial cells, tumor cells can be produced without using hTERT gene that has been conventionally required to produce tumor cells. In addition, the present invention also enables the preparation of a human breast cancer model, and particularly a triple negative breast cancer model, that are extremely similar to breast cancer tissue isolatedfrom human breast cancer patients.

INDUSTRIAL APPLICABILITY

According to the present invention, tumor cells can be provided that exhibit pathological characteristics similar to those of human breast cancer cells. These tumor cells have the potential to be useful in research on the biochemical mechanism of breast cancer, identification of target molecules of breast cancer treatment, and screening and testing of antitumor agents.

Sequence Listing Free Text

SEQ ID NO: 7: Synthetic DNA
SEQ ID NO: 8: Synthetic DNA

## Claims

1. An in vitro method for producing tumor cells by carrying out the following treatments on normal human mammary epithelial cells:

   (1) elimination of p53 function; and
   (2) introduction of a v-Src gene;

   wherein the tumor cells are negative for expression of estrogen receptor, negative for expression of progesterone receptor, and negative for expression of ERBB2.

2. The method according to claim 1, wherein introduction of a cyclin-dependent kinase gene is further carried out.

3. The method according to claim 2, wherein the cyclin-dependent kinase gene is a Cdk4 gene.

4. Tumor cells produced by the method according to any one of claims 1 to 3.

5. A method of screening anti-tumor agents, the method comprising:

   (a) contacting the tumor cells according to claim 4 with a candidate substance; and
   (b) detecting growth inhibitory effects on the tumor cells.

6. A cancer-bearing non-human animal model transplanted with the tumor cells according to claim 4.

7. The animal model according to claim 6, wherein the animal model is selected from mice, rats, pigs, dogs, monkeys, hamsters and rabbits.

**8.** A method of screening anti-tumor agents,
the method comprising:

(a) contacting the cancer-bearing animal model according to claim 6 or 7 with a candidate substance, and
(b) detecting growth inhibitory effects on tumor cells.

**Patentansprüche**

**1.** In-vitro-Verfahren zur Herstellung von Tumorzellen durch Anwenden der folgenden Behandlungen auf normale humane Brustdrüsenepithelzellen:

(1) Eliminierung der Funktion von p53, und
(2) Einführen eines v-Src-Gens,

wobei die Tumorzellen negativ für die Expression des Östrogenrezeptors, negativ für die Expression des Progesteronrezeptors, und negativ für die Expression von ERBB2 sind.

**2.** Verfahren gemäß Anspruch 1, wobei außerdem die Einführung eines Cyclin-abhängige-Kinase-Gens durchgeführt wird.

**3.** Verfahren gemäß Anspruch 2, wobei das Cyclin-abhängige-Kinase-Gen ein Cdk4-Gen ist.

**4.** Tumorzellen, hergestellt durch das Verfahren gemäß einem der Ansprüche 1 bis 3.

**5.** Verfahren zum Screening von Antitumoragenzien, wobei das Verfahren umfasst:

(a) Inkontaktbringen der Tumorzellen gemäß Anspruch 4 mit einer Kandidatensubstanz, und
(b) Detektieren der wachstumsinhibierenden Effekte auf die Tumorzellen.

**6.** Krebstragendes nicht-humanes Tiermodell, transplantiert mit den Tumorzellen gemäß Anspruch 4.

**7.** Tiermodell gemäß Anspruch 6, wobei das Tiermodell aus Mäusen, Ratten, Schweinen, Hunden, Affen, Hamstern und Kaninchen ausgewählt ist.

**8.** Verfahren zum Screening von Antitumoragenzien, wobei das Verfahren umfasst:

(a) Inkontaktbringen des krebstragenden Tiermodells gemäß Anspruch 6 oder 7 mit einer Kandidatensubstanz, und
(b) Detektieren von wachstumsinhibierenden Effekten auf Tumorzellen.

**Revendications**

**1.** Procédé in vitro de production de cellules tumorales, par mise en oeuvre, sur des cellules épithéliales mammaires humaines normales, des traitements suivants :

1) élimination de la fonction p53,
2) et introduction d'un gène v-Src,

les cellules tumorales étant négatives pour l'expression du récepteur des oestrogènes, négatives pour l'expression du récepteur de la progestérone et négatives pour l'expression de la protéine ERBB2.

**2.** Procédé conforme à la revendication 1, dans lequel est en outre mise en oeuvre l'introduction d'un gène de kinase cycline-dépendante.

**3.** Procédé conforme à la revendication 2, dans lequel le gène de kinase cycline-dépendante est un gène Cdk4.

**4.** Cellules tumorales produites selon un procédé conforme à l'une des revendications 1 à 3.

**5.** Procédé de recherche par criblage d'agents anti-tumoraux, lequel procédé comporte les étapes suivantes :

a) mettre des cellules tumorales, conformes à la revendication 4, en contact avec une substance candidate,
b) et détecter des effets d'inhibition de prolifération sur ces cellules tumorales.

**6.** Modèle animal non-humain qui subi une transplantation de cellules tumorales conformes à la revendication 4.

**7.** Modèle animal conforme à la revendication 6, lequel modèle animal est choisi parmi les souris, rats, porcs, chiens, singes, hamsters et lapins.

**8.** Procédé de recherche par criblage d'agents anti-tumoraux, lequel procédé comporte les étapes suivantes :

a) mettre un modèle animal de cancer, conforme à la revendication 6 ou 7, en contact avec une substance candidate,
b) et détecter des effets d'inhibition de prolifération sur ces cellules tumorales.

# Figure 1

# Figure 2

# Figure 3

## Western-blot analysis of HMEC derived tumorigenic cells

A
Anti-Phosphorylated-Src
(Tyr416) antibody

B
Anti-p53 antibody

C

Anti-Phosphorylated tyrosine
antibody (4G10)

1. HME/53 Cell
2. HME Cell
3. HME/53 Cell
4. HME/53/v-Src Cell

# Figure 4

**A**

**B**

MDA-MB 231 - 8 Weeks          HME/53/v-Src Cell - 3 Weeks

# Figure 5

**Tissue Immunostaining**

CK5/6

PgR

HE

ER

HER2

HME/53/v-Src Cell Xenograft (SC)

# Figure 6

HE X20

HER2 X20

PgR X20

ER X20

HME/53/Cdk4/v-Src Cell
Xenograft (SC)

**Figure 7**

| Genes Introduced | Rate of Tumorigenesis |
|---|---|
| p53CT | 0/2 |
| p53CT+ v-Src | 4/4 |
| p53CT+Cdk4 | 0/2 |
| p53CT+Cdk4 + v-Src | 4/4 |
| p53CT+ c-Myc | 0/4 |
| p53CT+ EGFR $^{T790M, L858R}$ | 0/4 |
| p53CT+ EGFR $^{T790M, L858R}$+c-Myc | 4/4 |

p53CT: DOMINANT NEGATIVE TP53 MUTANT

# Figure 8

HME/ 53/ EGFR $^{T790M.L858R}$/ c-Myc Cell Xenograft (SC)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20060222590 A1
- JP 2010154744 A

### Non-patent literature cited in the description

- **ELENBAAS, B. et al.** *Genes Dev.,* 2001, vol. 15, 50-65
- **KENDALL ; S. DISEAN.** *Cancer Res.,* 01 November 2005, vol. 65 (21), 9824-9828
- **ZHAO, J.J.** *Proc. Natl. Acad. Sci.,* 2006, vol. 31; 103 (44), 16296-16300
- **LANE, D.P.** *Nature,* 1992, vol. 358, 15-16
- **SATO, M. et al.** *Cancer Res.,* 2006, vol. 66, 2116-2128
- **SHAULIAN, E. et al.** *Mol. Cell. Biol.,* December 1992, vol. 12, 5581
- **MAYER, B.J. et al.** *J. Virol.,* December 1986, vol. 60 (3), 858-67
- **ZUO, L. et al.** *Nature Genet.,* 1996, vol. 12, 97-99
- **SHARMA, S.V et al.** *Nature Rev. Cancer,* 2007, vol. 7 (3), 169-81
- **MCCOY, M.S. et al.** *Mol. Cell. Biol.,* 1984, vol. 4, 1577-1582
- **SAMUELS, Y. et al.** *Science,* 2004, vol. 304, 554
- **SCOTT, K.D. et al.** *Cancer Res.,* 2007, vol. 67, 5622-5627
- **DOMINGUEZ-SOLA, D. et al.** *Nature,* 2007, vol. 448, 445-451
- **SAMBROOK ; RUSSELL.** Molecular Cloning: A Laboratory Manual. Cold Spring Harbor Laboratory Press, 2001, vol. 3
- **TANAKA, S. et al.** *Proc. Natl. Acad. Sci.,* 1997, vol. 94, 2356-2361
- *Genetic Induction of Tumorigenesis in Swine, Oncogene,* 11 September 2006, vol. 26, 1038-1045
- **AKAGI, T. et al.** *Mol. Cell. Biol.,* 2002, vol. 22, 7015-7023
- **SASAI, K. et al.** *Am. J. Surg. Pathol.,* 2008, vol. 32, 1220-1227
- Entellan Neu Reagent. Merck & Co,

SEQUENCE LISTING

<110> EISAI R&D MANAGEMENT CO., LTD.

<120> A METHOD OF PRODUCING A TUMOUR CELL FROM A NORMAL HUMAN MAMMARY GLAND EPITHELIAL CELL

<130> PCT11-0026

<150> JP 2010-154744
<151> 2010-07-07

<160> 10

<170> PatentIn version 3.4

<210> 1
<211> 312
<212> DNA
<213> Homo sapiens

<220>
<221> CDS
<222> (1)..(312)

<400> 1

```
atg tac cca tac gat gtt cca gat tac gct cca ggg agc act aag cga      48
Met Tyr Pro Tyr Asp Val Pro Asp Tyr Ala Pro Gly Ser Thr Lys Arg
1               5                   10                  15

gca ctg ccc aac aac acc agc tcc tct ccc cag cca aag aag aaa cca      96
Ala Leu Pro Asn Asn Thr Ser Ser Ser Pro Gln Pro Lys Lys Lys Pro
                20                  25                  30

ctg gat gga gaa tat ttc acc ctt cag atc cgt ggg cgt gag cgc ttc     144
Leu Asp Gly Glu Tyr Phe Thr Leu Gln Ile Arg Gly Arg Glu Arg Phe
            35                  40                  45

gag atg ttc cga gag ctg aat gag gcc ttg gaa ctc aag gat gcc cag     192
Glu Met Phe Arg Glu Leu Asn Glu Ala Leu Glu Leu Lys Asp Ala Gln
        50                  55                  60

gct ggg aag gag cca ggg ggg agc agg gct cac tcc agc cac ctg aag     240
Ala Gly Lys Glu Pro Gly Gly Ser Arg Ala His Ser Ser His Leu Lys
65                  70                  75                  80

tcc aaa aag ggt cag tct acc tcc cgc cat aaa aaa ctc atg ttc aag     288
Ser Lys Lys Gly Gln Ser Thr Ser Arg His Lys Lys Leu Met Phe Lys
                85                  90                  95

aca gaa ggg cct gac tca gac tga                                     312
Thr Glu Gly Pro Asp Ser Asp
                100
```

<210> 2
<211> 103

<212> PRT
<213> Homo sapiens

<400> 2

```
        Met Tyr Pro Tyr Asp Val Pro Asp Tyr Ala Pro Gly Ser Thr Lys Arg
        1               5                   10                  15

        Ala Leu Pro Asn Asn Thr Ser Ser Ser Pro Gln Pro Lys Lys Lys Pro
                        20                  25                  30

        Leu Asp Gly Glu Tyr Phe Thr Leu Gln Ile Arg Gly Arg Glu Arg Phe
                    35                  40                  45

        Glu Met Phe Arg Glu Leu Asn Glu Ala Leu Glu Leu Lys Asp Ala Gln
                50                  55                  60

        Ala Gly Lys Glu Pro Gly Gly Ser Arg Ala His Ser Ser His Leu Lys
        65                  70                  75                  80

        Ser Lys Lys Gly Gln Ser Thr Ser Arg His Lys Lys Leu Met Phe Lys
                        85                  90                  95

        Thr Glu Gly Pro Asp Ser Asp
                        100
```

<210> 3
<211> 1581
<212> DNA
<213> Rous sarcoma virus

<220>
<221> CDS
<222> (1)..(1581)

<400> 3

```
atg ggg agt agc aag agc aag cct aag gac ccc agc cag cgc cgg cgc          48
Met Gly Ser Ser Lys Ser Lys Pro Lys Asp Pro Ser Gln Arg Arg Arg
1               5                   10                  15

agc ctg gag cca ccc gac agc acc cac cac ggg gga ttc cca gcc tcg          96
Ser Leu Glu Pro Pro Asp Ser Thr His His Gly Gly Phe Pro Ala Ser
                20                  25                  30

cag acc ccc aac aag aca gca gcc ccc gac acg cac cgc acc ccc agc         144
Gln Thr Pro Asn Lys Thr Ala Ala Pro Asp Thr His Arg Thr Pro Ser
        35                  40                  45

cgc tcc ttc ggg acc gtg gcc acc gag ccc aag ctc ttc ggg ggc ttc        192
Arg Ser Phe Gly Thr Val Ala Thr Glu Pro Lys Leu Phe Gly Gly Phe
        50                  55                  60

aac act tct gac acc gtt acg tcg ccg cag cgt gcc ggg gca ctg gct        240
Asn Thr Ser Asp Thr Val Thr Ser Pro Gln Arg Ala Gly Ala Leu Ala
65                  70                  75                  80

ggc ggc gtc acc act ttc gtg gct ctc tac gac tac gag tcc tgg att        288
Gly Gly Val Thr Thr Phe Val Ala Leu Tyr Asp Tyr Glu Ser Trp Ile
                85                  90                  95

gaa acg gac ttg tcc ttc aag aaa gga gaa cgg ctg cag att gtc aac        336
Glu Thr Asp Leu Ser Phe Lys Lys Gly Glu Arg Leu Gln Ile Val Asn
                100                 105                 110
```

```
aac acg gaa ggt aac tgg tgg ctg gct cat tcc ctc act aca gga cag       384
Asn Thr Glu Gly Asn Trp Trp Leu Ala His Ser Leu Thr Thr Gly Gln
        115             120             125

acg ggc tac atc ccc agt aac tat gtc gcg ccc tca gac tcc atc cag       432
Thr Gly Tyr Ile Pro Ser Asn Tyr Val Ala Pro Ser Asp Ser Ile Gln
        130             135             140

gct gaa gag tgg tac ttt ggg aag atc act cgt cgg gag tcc gag cgg       480
Ala Glu Glu Trp Tyr Phe Gly Lys Ile Thr Arg Arg Glu Ser Glu Arg
145             150             155             160

ctg ctg ctc aac ccc gaa aac ccc cgg gga acc ttc ttg gtc cgg gag       528
Leu Leu Leu Asn Pro Glu Asn Pro Arg Gly Thr Phe Leu Val Arg Glu
                165             170             175

agc gag acg aca aaa ggt gcc tat tgc ctc tcc gtt tct gac ttt gac       576
Ser Glu Thr Thr Lys Gly Ala Tyr Cys Leu Ser Val Ser Asp Phe Asp
            180             185             190

aac gcc aag ggg ctc aat gtg aag cac tac aag atc cgc aag ctg gac       624
Asn Ala Lys Gly Leu Asn Val Lys His Tyr Lys Ile Arg Lys Leu Asp
            195             200             205

agc ggc ggc ttc tac atc acc tca cgc aca cag ttc agc agc ctg cag       672
Ser Gly Gly Phe Tyr Ile Thr Ser Arg Thr Gln Phe Ser Ser Leu Gln
            210             215             220

cag ctg gtg gcc tac tac tcc aaa cat gct gat ggc ttg tgc cac cgc       720
Gln Leu Val Ala Tyr Tyr Ser Lys His Ala Asp Gly Leu Cys His Arg
225             230             235             240

ctg acc aac gtc tgc ccc acg tcc aag ccc cag acc cag gga ctc gcc       768
Leu Thr Asn Val Cys Pro Thr Ser Lys Pro Gln Thr Gln Gly Leu Ala
                245             250             255

aag gac gcg tgg gaa atc ccc cgg gag tcg ctg cgg ctg gag gtg aag       816
Lys Asp Ala Trp Glu Ile Pro Arg Glu Ser Leu Arg Leu Glu Val Lys
                260             265             270

ctg ggg cag ggc tgc ttt gga gag gtc tgg atg ggg acc tgg aac ggc       864
Leu Gly Gln Gly Cys Phe Gly Glu Val Trp Met Gly Thr Trp Asn Gly
            275             280             285

acc acc aga gtg gcc ata aag act ctg aag ccc ggc acc atg tcc ccg       912
Thr Thr Arg Val Ala Ile Lys Thr Leu Lys Pro Gly Thr Met Ser Pro
        290             295             300

gag gcc ttc ctg cag gaa gcc caa gtg atg aag aag ctc cgg cat gag       960
Glu Ala Phe Leu Gln Glu Ala Gln Val Met Lys Lys Leu Arg His Glu
305             310             315             320

aag ctg gtt caa ctg tac gca gtg gtg tcg gaa gag ccc atc tac atc      1008
Lys Leu Val Gln Leu Tyr Ala Val Val Ser Glu Glu Pro Ile Tyr Ile
                325             330             335

gtc att gag tac atg agc aag ggg agc ctc ctg gat ttc ctg aag gga      1056
Val Ile Glu Tyr Met Ser Lys Gly Ser Leu Leu Asp Phe Leu Lys Gly
            340             345             350

gag atg ggc aag tac ctg cgg ctg cca cag ctc gtt gat atg gct gct      1104
Glu Met Gly Lys Tyr Leu Arg Leu Pro Gln Leu Val Asp Met Ala Ala
            355             360             365

cag att gca tcc ggc atg gcc tat gtg gag agg atg aac tac gtg cac      1152
```

```
Gln Ile Ala Ser Gly Met Ala Tyr Val Glu Arg Met Asn Tyr Val His
    370             375             380

cga gac ctg cgg gcg gcc aac atc ctg gtg ggg gag aac ctg gtg tgc      1200
Arg Asp Leu Arg Ala Ala Asn Ile Leu Val Gly Glu Asn Leu Val Cys
385             390             395             400

aag gtg gct gac ttt ggg ctg gca cgc ctc atc gag gac aac gag tac      1248
Lys Val Ala Asp Phe Gly Leu Ala Arg Leu Ile Glu Asp Asn Glu Tyr
            405             410             415

aca gca cgg caa ggt gcc aag ttc ccc atc aag tgg aca gcc ccc gag      1296
Thr Ala Arg Gln Gly Ala Lys Phe Pro Ile Lys Trp Thr Ala Pro Glu
            420             425             430

gca gcc ctc tat ggc cgg ttc acc atc aag tcg gat gtc tgg tcc ttc      1344
Ala Ala Leu Tyr Gly Arg Phe Thr Ile Lys Ser Asp Val Trp Ser Phe
            435             440             445

ggc atc ctg ctg act gag ctg acc acc aag ggc cgg gtg cca tac cca      1392
Gly Ile Leu Leu Thr Glu Leu Thr Thr Lys Gly Arg Val Pro Tyr Pro
450             455             460

ggg atg ggc aac ggg gag gtg ctg gac cgg gtg gag agg ggc tac cgc      1440
Gly Met Gly Asn Gly Glu Val Leu Asp Arg Val Glu Arg Gly Tyr Arg
465             470             475             480

atg ccc tgc ccg ccc gag tgc ccc gag tcg ctg cat gac ctt atg tgc      1488
Met Pro Cys Pro Pro Glu Cys Pro Glu Ser Leu His Asp Leu Met Cys
            485             490             495

cag tgc tgg cgg agg gac cct gag gag cgg ccc act ttc gag tac ctg      1536
Gln Cys Trp Arg Arg Asp Pro Glu Glu Arg Pro Thr Phe Glu Tyr Leu
            500             505             510

cag gcc cag ctg ctc cct gct tgt gtg ttg gag gtc gct gag tag         1581
Gln Ala Gln Leu Leu Pro Ala Cys Val Leu Glu Val Ala Glu
            515             520             525
```

<210> 4
<211> 526
<212> PRT
<213> Rous sarcoma virus

<400> 4

```
Met Gly Ser Ser Lys Ser Lys Pro Lys Asp Pro Ser Gln Arg Arg Arg
1               5                   10                  15

Ser Leu Glu Pro Pro Asp Ser Thr His His Gly Gly Phe Pro Ala Ser
            20                  25                  30

Gln Thr Pro Asn Lys Thr Ala Ala Pro Asp Thr His Arg Thr Pro Ser
            35                  40                  45

Arg Ser Phe Gly Thr Val Ala Thr Glu Pro Lys Leu Phe Gly Gly Phe
    50                  55                  60

Asn Thr Ser Asp Thr Val Thr Ser Pro Gln Arg Ala Gly Ala Leu Ala
65                  70                  75                  80
```

```
Gly Gly Val Thr Thr Phe Val Ala Leu Tyr Asp Tyr Glu Ser Trp Ile
            85                  90                  95

Glu Thr Asp Leu Ser Phe Lys Lys Gly Glu Arg Leu Gln Ile Val Asn
            100                 105                 110

Asn Thr Glu Gly Asn Trp Trp Leu Ala His Ser Leu Thr Thr Gly Gln
            115                 120                 125

Thr Gly Tyr Ile Pro Ser Asn Tyr Val Ala Pro Ser Asp Ser Ile Gln
    130                 135                 140

Ala Glu Glu Trp Tyr Phe Gly Lys Ile Thr Arg Arg Glu Ser Glu Arg
145                 150                 155                 160

Leu Leu Leu Asn Pro Glu Asn Pro Arg Gly Thr Phe Leu Val Arg Glu
                165                 170                 175

Ser Glu Thr Thr Lys Gly Ala Tyr Cys Leu Ser Val Ser Asp Phe Asp
            180                 185                 190

Asn Ala Lys Gly Leu Asn Val Lys His Tyr Lys Ile Arg Lys Leu Asp
            195                 200                 205

Ser Gly Gly Phe Tyr Ile Thr Ser Arg Thr Gln Phe Ser Ser Leu Gln
    210                 215                 220

Gln Leu Val Ala Tyr Tyr Ser Lys His Ala Asp Gly Leu Cys His Arg
225                 230                 235                 240

Leu Thr Asn Val Cys Pro Thr Ser Lys Pro Gln Thr Gln Gly Leu Ala
                245                 250                 255

Lys Asp Ala Trp Glu Ile Pro Arg Glu Ser Leu Arg Leu Glu Val Lys
                260                 265                 270

Leu Gly Gln Gly Cys Phe Gly Glu Val Trp Met Gly Thr Trp Asn Gly
            275                 280                 285

Thr Thr Arg Val Ala Ile Lys Thr Leu Lys Pro Gly Thr Met Ser Pro
    290                 295                 300

Glu Ala Phe Leu Gln Glu Ala Gln Val Met Lys Lys Leu Arg His Glu
305                 310                 315                 320

Lys Leu Val Gln Leu Tyr Ala Val Val Ser Glu Glu Pro Ile Tyr Ile
                325                 330                 335
```

```
Val Ile Glu Tyr Met Ser Lys Gly Ser Leu Leu Asp Phe Leu Lys Gly
        340             345                 350

Glu Met Gly Lys Tyr Leu Arg Leu Pro Gln Leu Val Asp Met Ala Ala
        355             360                 365

Gln Ile Ala Ser Gly Met Ala Tyr Val Glu Arg Met Asn Tyr Val His
        370             375                 380

Arg Asp Leu Arg Ala Ala Asn Ile Leu Val Gly Glu Asn Leu Val Cys
385             390                 395                 400

Lys Val Ala Asp Phe Gly Leu Ala Arg Leu Ile Glu Asp Asn Glu Tyr
                405                 410                 415

Thr Ala Arg Gln Gly Ala Lys Phe Pro Ile Lys Trp Thr Ala Pro Glu
        420             425                 430

Ala Ala Leu Tyr Gly Arg Phe Thr Ile Lys Ser Asp Val Trp Ser Phe
        435             440                 445

Gly Ile Leu Leu Thr Glu Leu Thr Thr Lys Gly Arg Val Pro Tyr Pro
    450             455                 460

Gly Met Gly Asn Gly Glu Val Leu Asp Arg Val Glu Arg Gly Tyr Arg
465             470                 475                 480

Met Pro Cys Pro Pro Glu Cys Pro Glu Ser Leu His Asp Leu Met Cys
                485                 490                 495

Gln Cys Trp Arg Arg Asp Pro Glu Glu Arg Pro Thr Phe Glu Tyr Leu
        500             505                 510

Gln Ala Gln Leu Leu Pro Ala Cys Val Leu Glu Val Ala Glu
        515             520                 525
```

<210> 5
<211> 912
<212> DNA
<213> Homo sapiens

<220>
<221> CDS
<222> (1)..(912)

<400> 5

```
atg gct acc tct cga tat gag cca gtg gct gaa att ggt gtc ggt gcc      48
Met Ala Thr Ser Arg Tyr Glu Pro Val Ala Glu Ile Gly Val Gly Ala
1                   5                   10                  15

tat ggg aca gtg tac aag gcc cgt gat ccc cac agt ggc cac ttt gtg      96
```

```
              Tyr Gly Thr Val Tyr Lys Ala Arg Asp Pro His Ser Gly His Phe Val
                      20                  25                  30

gcc ctc aag agt gtg aga gtc ccc aat gga gga gga ggt gga gga ggc          144
Ala Leu Lys Ser Val Arg Val Pro Asn Gly Gly Gly Gly Gly Gly Gly
        35                  40                  45

ctt ccc atc agc aca gtt cgt gag gtg gct tta ctg agg cga ctg gag          192
Leu Pro Ile Ser Thr Val Arg Glu Val Ala Leu Leu Arg Arg Leu Glu
        50                  55                  60

gct ttt gag cat ccc aat gtt gtc cgg ctg atg gac gtc tgt gcc aca          240
Ala Phe Glu His Pro Asn Val Val Arg Leu Met Asp Val Cys Ala Thr
65                  70                  75                  80

tcc cga act gac cgg gag atc aag gta acc ctg gtg ttt gag cat gta          288
Ser Arg Thr Asp Arg Glu Ile Lys Val Thr Leu Val Phe Glu His Val
                85                  90                  95

gac cag gac cta agg aca tat ctg gac aag gca ccc cca cca ggc ttg          336
Asp Gln Asp Leu Arg Thr Tyr Leu Asp Lys Ala Pro Pro Pro Gly Leu
                100                 105                 110

cca gcc gaa acg atc aag gat ctg atg cgc cag ttt cta aga ggc cta          384
Pro Ala Glu Thr Ile Lys Asp Leu Met Arg Gln Phe Leu Arg Gly Leu
        115                 120                 125

gat ttc ctt cat gcc aat tgc atc gtt cac cga gat ctg aag cca gag          432
Asp Phe Leu His Ala Asn Cys Ile Val His Arg Asp Leu Lys Pro Glu
        130                 135                 140

aac att ctg gtg aca agt ggt gga aca gtc aag ctg gct gac ttt ggc          480
Asn Ile Leu Val Thr Ser Gly Gly Thr Val Lys Leu Ala Asp Phe Gly
145                 150                 155                 160

ctg gcc aga atc tac agc tac cag atg gca ctt aca ccc gtg gtt gtt          528
Leu Ala Arg Ile Tyr Ser Tyr Gln Met Ala Leu Thr Pro Val Val Val
                165                 170                 175

aca ctc tgg tac cga gct ccc gaa gtt ctt ctg cag tcc aca tat gca          576
Thr Leu Trp Tyr Arg Ala Pro Glu Val Leu Leu Gln Ser Thr Tyr Ala
                180                 185                 190

aca cct gtg gac atg tgg agt gtt ggc tgt atc ttt gca gag atg ttt          624
Thr Pro Val Asp Met Trp Ser Val Gly Cys Ile Phe Ala Glu Met Phe
        195                 200                 205

cgt cga aag cct ctc ttc tgt gga aac tct gaa gcc gac cag ttg ggc          672
Arg Arg Lys Pro Leu Phe Cys Gly Asn Ser Glu Ala Asp Gln Leu Gly
        210                 215                 220

aaa atc ttt gac ctg att ggg ctg cct cca gag gat gac tgg cct cga          720
Lys Ile Phe Asp Leu Ile Gly Leu Pro Pro Glu Asp Asp Trp Pro Arg
225                 230                 235                 240

gat gta tcc ctg ccc cgt gga gcc ttt ccc ccc aga ggg ccc cgc cca          768
Asp Val Ser Leu Pro Arg Gly Ala Phe Pro Pro Arg Gly Pro Arg Pro
                245                 250                 255

gtg cag tcg gtg gta cct gag atg gag gag tcg gga gca cag ctg ctg          816
Val Gln Ser Val Val Pro Glu Met Glu Glu Ser Gly Ala Gln Leu Leu
                260                 265                 270

ctg gaa atg ctg act ttt aac cca cac aag cga atc tct gcc ttt cga          864
Leu Glu Met Leu Thr Phe Asn Pro His Lys Arg Ile Ser Ala Phe Arg
```

```
                 275                  280                       285

        gct ctg cag cac tct tat cta cat aag gat gaa ggt aat ccg gag tga        912
        Ala Leu Gln His Ser Tyr Leu His Lys Asp Glu Gly Asn Pro Glu
            290                  295                  300
```

<210> 6
<211> 303
<212> PRT
<213> Homo sapiens

<400> 6

```
Met Ala Thr Ser Arg Tyr Glu Pro Val Ala Glu Ile Gly Val Gly Ala
1               5               10              15

Tyr Gly Thr Val Tyr Lys Ala Arg Asp Pro His Ser Gly His Phe Val
        20              25              30

Ala Leu Lys Ser Val Arg Val Pro Asn Gly Gly Gly Gly Gly Gly Gly
        35              40              45

Leu Pro Ile Ser Thr Val Arg Glu Val Ala Leu Leu Arg Arg Leu Glu
    50              55              60

Ala Phe Glu His Pro Asn Val Val Arg Leu Met Asp Val Cys Ala Thr
65              70              75              80

Ser Arg Thr Asp Arg Glu Ile Lys Val Thr Leu Val Phe Glu His Val
            85              90              95

Asp Gln Asp Leu Arg Thr Tyr Leu Asp Lys Ala Pro Pro Pro Gly Leu
        100             105             110

Pro Ala Glu Thr Ile Lys Asp Leu Met Arg Gln Phe Leu Arg Gly Leu
        115             120             125

Asp Phe Leu His Ala Asn Cys Ile Val His Arg Asp Leu Lys Pro Glu
        130             135             140

Asn Ile Leu Val Thr Ser Gly Gly Thr Val Lys Leu Ala Asp Phe Gly
145             150             155             160

Leu Ala Arg Ile Tyr Ser Tyr Gln Met Ala Leu Thr Pro Val Val Val
        165             170             175

Thr Leu Trp Tyr Arg Ala Pro Glu Val Leu Leu Gln Ser Thr Tyr Ala
        180             185             190

Thr Pro Val Asp Met Trp Ser Val Gly Cys Ile Phe Ala Glu Met Phe
        195             200             205
```

```
Arg Arg Lys Pro Leu Phe Cys Gly Asn Ser Glu Ala Asp Gln Leu Gly
    210                 215                 220

Lys Ile Phe Asp Leu Ile Gly Leu Pro Pro Glu Asp Asp Trp Pro Arg
225                 230                 235                 240

Asp Val Ser Leu Pro Arg Gly Ala Phe Pro Pro Arg Gly Pro Arg Pro
                245                 250                 255

Val Gln Ser Val Val Pro Glu Met Glu Glu Ser Gly Ala Gln Leu Leu
            260                 265                 270

Leu Glu Met Leu Thr Phe Asn Pro His Lys Arg Ile Ser Ala Phe Arg
            275                 280                 285

Ala Leu Gln His Ser Tyr Leu His Lys Asp Glu Gly Asn Pro Glu
    290                 295                 300
```

<210> 7
<211> 20
<212> DNA
<213> Artificial

<220>
<223> Synthetic DNA

<400> 7
gatcctcccc agtgagaagt          20

<210> 8
<211> 21
<212> DNA
<213> Artificial

<220>
<223> Synthetic DNA

<400> 8
ctcactagcc atctggagta g          21

<210> 9
<211> 3633
<212> DNA
<213> Homo sapiens

<220>
<221> CDS
<222> (1)..(3633)

<400> 9

```
atg cga ccc tcc ggg acg gcc ggg gca gcg ctc ctg gcg ctg ctg gct        48
Met Arg Pro Ser Gly Thr Ala Gly Ala Ala Leu Leu Ala Leu Leu Ala
1               5                   10                  15


gcg ctc tgc ccg gcg agt cgg gct ctg gag gaa aag aaa gtt tgc caa        96
```

EP 2 592 141 B1

```
Ala Leu Cys Pro Ala Ser Arg Ala Leu Glu Glu Lys Lys Val Cys Gln
        20              25                  30

ggc acg agt aac aag ctc acg cag ttg ggc act ttt gaa gat cat ttt    144
Gly Thr Ser Asn Lys Leu Thr Gln Leu Gly Thr Phe Glu Asp His Phe
        35              40                  45

ctc agc ctc cag agg atg ttc aat aac tgt gag gtg gtc ctt ggg aat    192
Leu Ser Leu Gln Arg Met Phe Asn Asn Cys Glu Val Val Leu Gly Asn
        50              55                  60

ttg gaa att acc tat gtg cag agg aat tat gat ctt tcc ttc tta aag    240
Leu Glu Ile Thr Tyr Val Gln Arg Asn Tyr Asp Leu Ser Phe Leu Lys
65              70                  75                  80

acc atc cag gag gtg gct ggt tat gtc ctc att gcc ctc aac aca gtg    288
Thr Ile Gln Glu Val Ala Gly Tyr Val Leu Ile Ala Leu Asn Thr Val
                85              90                  95

gag cga att cct ttg gaa aac ctg cag atc atc aga gga aat atg tac    336
Glu Arg Ile Pro Leu Glu Asn Leu Gln Ile Ile Arg Gly Asn Met Tyr
                100             105                 110

tac gaa aat tcc tat gcc tta gca gtc tta tct aac tat gat gca aat    384
Tyr Glu Asn Ser Tyr Ala Leu Ala Val Leu Ser Asn Tyr Asp Ala Asn
            115             120                 125

aaa acc gga ctg aag gag ctg ccc atg aga aat tta cag gaa atc ctg    432
Lys Thr Gly Leu Lys Glu Leu Pro Met Arg Asn Leu Gln Glu Ile Leu
        130             135                 140

cat ggc gcc gtg cgg ttc agc aac aac cct gcc ctg tgc aac gtg gag    480
His Gly Ala Val Arg Phe Ser Asn Asn Pro Ala Leu Cys Asn Val Glu
145                 150                 155                 160

agc atc cag tgg cgg gac ata gtc agc agt gac ttt ctc agc aac atg    528
Ser Ile Gln Trp Arg Asp Ile Val Ser Ser Asp Phe Leu Ser Asn Met
                165                 170                 175

tcg atg gac ttc cag aac cac ctg ggc agc tgc caa aag tgt gat cca    576
Ser Met Asp Phe Gln Asn His Leu Gly Ser Cys Gln Lys Cys Asp Pro
            180                 185                 190

agc tgt ccc aat ggg agc tgc tgg ggt gca gga gag gag aac tgc cag    624
Ser Cys Pro Asn Gly Ser Cys Trp Gly Ala Gly Glu Glu Asn Cys Gln
        195                 200                 205

aaa ctg acc aaa atc atc tgt gcc cag cag tgc tcc ggg cgc tgc cgt    672
Lys Leu Thr Lys Ile Ile Cys Ala Gln Gln Cys Ser Gly Arg Cys Arg
        210                 215                 220

ggc aag tcc ccc agt gac tgc tgc cac aac cag tgt gct gca ggc tgc    720
Gly Lys Ser Pro Ser Asp Cys Cys His Asn Gln Cys Ala Ala Gly Cys
225                 230                 235                 240

aca ggc ccc cgg gag agc gac tgc ctg gtc tgc cgc aaa ttc cga gac    768
Thr Gly Pro Arg Glu Ser Asp Cys Leu Val Cys Arg Lys Phe Arg Asp
                245                 250                 255

gaa gcc acg tgc aag gac acc tgc ccc cca ctc atg ctc tac aac ccc    816
Glu Ala Thr Cys Lys Asp Thr Cys Pro Pro Leu Met Leu Tyr Asn Pro
            260                 265                 270

acc acg tac cag atg gat gtg aac ccc gag ggc aaa tac agc ttt ggt    864
Thr Thr Tyr Gln Met Asp Val Asn Pro Glu Gly Lys Tyr Ser Phe Gly
```

27

```
                    275                    280                    285

         gcc acc tgc gtg aag aag tgt ccc cgt aat tat gtg gtg aca gat cac       912
         Ala Thr Cys Val Lys Lys Cys Pro Arg Asn Tyr Val Val Thr Asp His
             290                 295                 300

         ggc tcg tgc gtc cga gcc tgt ggg gcc gac agc tat gag atg gag gaa       960
         Gly Ser Cys Val Arg Ala Cys Gly Ala Asp Ser Tyr Glu Met Glu Glu
         305                 310                 315                 320

         gac ggc gtc cgc aag tgt aag aag tgc gaa ggg cct tgc cgc aaa gtg      1008
         Asp Gly Val Arg Lys Cys Lys Lys Cys Glu Gly Pro Cys Arg Lys Val
                         325                 330                 335

         tgt aac gga ata ggt att ggt gaa ttt aaa gac tca ctc tcc ata aat      1056
         Cys Asn Gly Ile Gly Ile Gly Glu Phe Lys Asp Ser Leu Ser Ile Asn
                     340                 345                 350

         gct acg aat att aaa cac ttc aaa aac tgc acc tcc atc agt ggc gat      1104
         Ala Thr Asn Ile Lys His Phe Lys Asn Cys Thr Ser Ile Ser Gly Asp
                 355                 360                 365

         ctc cac atc ctg ccg gtg gca ttt agg ggt gac tcc ttc aca cat act      1152
         Leu His Ile Leu Pro Val Ala Phe Arg Gly Asp Ser Phe Thr His Thr
         370                 375                 380

         cct cct ctg gat cca cag gaa ctg gat att ctg aaa acc gta aag gaa      1200
         Pro Pro Leu Asp Pro Gln Glu Leu Asp Ile Leu Lys Thr Val Lys Glu
         385                 390                 395                 400

         atc aca ggg ttt ttg ctg att cag gct tgg cct gaa aac agg acg gac      1248
         Ile Thr Gly Phe Leu Leu Ile Gln Ala Trp Pro Glu Asn Arg Thr Asp
                         405                 410                 415

         ctc cat gcc ttt gag aac cta gaa atc ata cgc ggc agg acc aag caa      1296
         Leu His Ala Phe Glu Asn Leu Glu Ile Ile Arg Gly Arg Thr Lys Gln
                     420                 425                 430

         cat ggt cag ttt tct ctt gca gtc gtc agc ctg aac ata aca tcc ttg      1344
         His Gly Gln Phe Ser Leu Ala Val Val Ser Leu Asn Ile Thr Ser Leu
                     435                 440                 445

         gga tta cgc tcc ctc aag gag ata agt gat gga gat gtg ata att tca      1392
         Gly Leu Arg Ser Leu Lys Glu Ile Ser Asp Gly Asp Val Ile Ile Ser
                 450                 455                 460

         gga aac aaa aat ttg tgc tat gca aat aca ata aac tgg aaa aaa ctg      1440
         Gly Asn Lys Asn Leu Cys Tyr Ala Asn Thr Ile Asn Trp Lys Lys Leu
         465                 470                 475                 480

         ttt ggg acc tcc ggt cag aaa acc aaa att ata agc aac aga ggt gaa      1488
         Phe Gly Thr Ser Gly Gln Lys Thr Lys Ile Ile Ser Asn Arg Gly Glu
                         485                 490                 495

         aac agc tgc aag gcc aca ggc cag gtc tgc cat gcc ttg tgc tcc ccc      1536
         Asn Ser Cys Lys Ala Thr Gly Gln Val Cys His Ala Leu Cys Ser Pro
                     500                 505                 510

         gag ggc tgc tgg ggc ccg gag ccc agg gac tgc gtc tct tgc cgg aat      1584
         Glu Gly Cys Trp Gly Pro Glu Pro Arg Asp Cys Val Ser Cys Arg Asn
                     515                 520                 525

         gtc agc cga ggc agg gaa tgc gtg gac aag tgc aac ctt ctg gag ggt      1632
         Val Ser Arg Gly Arg Glu Cys Val Asp Lys Cys Asn Leu Leu Glu Gly
                 530                 535                 540
```

```
gag cca agg gag ttt gtg gag aac tct gag tgc ata cag tgc cac cca      1680
Glu Pro Arg Glu Phe Val Glu Asn Ser Glu Cys Ile Gln Cys His Pro
545             550             555             560

gag tgc ctg cct cag gcc atg aac atc acc tgc aca gga cgg gga cca      1728
Glu Cys Leu Pro Gln Ala Met Asn Ile Thr Cys Thr Gly Arg Gly Pro
            565             570             575

gac aac tgt atc cag tgt gcc cac tac att gac ggc ccc cac tgc gtc      1776
Asp Asn Cys Ile Gln Cys Ala His Tyr Ile Asp Gly Pro His Cys Val
            580             585             590

aag acc tgc ccg gca gga gtc atg gga gaa aac aac acc ctg gtc tgg      1824
Lys Thr Cys Pro Ala Gly Val Met Gly Glu Asn Asn Thr Leu Val Trp
            595             600             605

aag tac gca gac gcc ggc cat gtg tgc cac ctg tgc cat cca aac tgc      1872
Lys Tyr Ala Asp Ala Gly His Val Cys His Leu Cys His Pro Asn Cys
            610             615             620

acc tac gga tgc act ggg cca ggt ctt gaa ggc tgt cca acg aat ggg      1920
Thr Tyr Gly Cys Thr Gly Pro Gly Leu Glu Gly Cys Pro Thr Asn Gly
625             630             635             640

cct aag atc ccg tcc atc gcc act ggg atg gtg ggg gcc ctc ctc ttg      1968
Pro Lys Ile Pro Ser Ile Ala Thr Gly Met Val Gly Ala Leu Leu Leu
            645             650             655

ctg ctg gtg gtg gcc ctg ggg atc ggc ctc ttc atg cga agg cgc cac      2016
Leu Leu Val Val Ala Leu Gly Ile Gly Leu Phe Met Arg Arg Arg His
            660             665             670

atc gtt cgg aag cgc acg ctg cgg agg ctg ctg cag gag agg gag ctt      2064
Ile Val Arg Lys Arg Thr Leu Arg Arg Leu Leu Gln Glu Arg Glu Leu
            675             680             685

gtg gag cct ctt aca ccc agt gga gaa gct ccc aac caa gct ctc ttg      2112
Val Glu Pro Leu Thr Pro Ser Gly Glu Ala Pro Asn Gln Ala Leu Leu
            690             695             700

agg atc ttg aag gaa act gaa ttc aaa aag atc aaa gtg ctg ggc tcc      2160
Arg Ile Leu Lys Glu Thr Glu Phe Lys Lys Ile Lys Val Leu Gly Ser
705             710             715             720

ggt gcg ttc ggc acg gtg tat aag gga ctc tgg atc cca gaa ggt gag      2208
Gly Ala Phe Gly Thr Val Tyr Lys Gly Leu Trp Ile Pro Glu Gly Glu
            725             730             735

aaa gtt aaa att ccc gtc gct atc aag gaa tta aga gaa gca aca tct      2256
Lys Val Lys Ile Pro Val Ala Ile Lys Glu Leu Arg Glu Ala Thr Ser
            740             745             750

ccg aaa gcc aac aag gaa atc ctc gat gaa gcc tac gtg atg gcc agc      2304
Pro Lys Ala Asn Lys Glu Ile Leu Asp Glu Ala Tyr Val Met Ala Ser
            755             760             765

gtg gac aac ccc cac gtg tgc cgc ctg ctg ggc atc tgc ctc acc tcc      2352
Val Asp Asn Pro His Val Cys Arg Leu Leu Gly Ile Cys Leu Thr Ser
            770             775             780

acc gtg cag ctc atc atg cag ctc atg ccc ttc ggc tgc ctc ctg gac      2400
Thr Val Gln Leu Ile Met Gln Leu Met Pro Phe Gly Cys Leu Leu Asp
785             790             795             800
```

```
tat gtc cgg gaa cac aaa gac aat att ggc tcc cag tac ctg ctc aac          2448
Tyr Val Arg Glu His Lys Asp Asn Ile Gly Ser Gln Tyr Leu Leu Asn
                805                 810                 815

tgg tgt gtg cag atc gca aag ggc atg aac tac ttg gag gac cgt cgc          2496
Trp Cys Val Gln Ile Ala Lys Gly Met Asn Tyr Leu Glu Asp Arg Arg
                820                 825                 830

ttg gtg cac cgc gac ctg gca gcc agg aac gta ctg gtg aaa aca ccg          2544
Leu Val His Arg Asp Leu Ala Ala Arg Asn Val Leu Val Lys Thr Pro
                835                 840                 845

cag cat gtc aag atc aca gat ttt ggg cgg gcc aaa ctg ctg ggt gcg          2592
Gln His Val Lys Ile Thr Asp Phe Gly Arg Ala Lys Leu Leu Gly Ala
                850                 855                 860

gaa gag aaa gaa tac cat gca gaa gga ggc aaa gtg cct atc aag tgg          2640
Glu Glu Lys Glu Tyr His Ala Glu Gly Gly Lys Val Pro Ile Lys Trp
865                 870                 875                 880

atg gca ttg gaa tca att tta cac aga atc tat acc cac cag agt gat          2688
Met Ala Leu Glu Ser Ile Leu His Arg Ile Tyr Thr His Gln Ser Asp
                885                 890                 895

gtc tgg agc tac ggg gtg acc gtt tgg gag ttg atg acc ttt gga tcc          2736
Val Trp Ser Tyr Gly Val Thr Val Trp Glu Leu Met Thr Phe Gly Ser
                900                 905                 910

aag cca tat gac gga atc cct gcc agc gag atc tcc tcc atc ctg gag          2784
Lys Pro Tyr Asp Gly Ile Pro Ala Ser Glu Ile Ser Ser Ile Leu Glu
                915                 920                 925

aaa gga gaa cgc ctc cct cag cca ccc ata tgt acc atc gat gtc tac          2832
Lys Gly Glu Arg Leu Pro Gln Pro Pro Ile Cys Thr Ile Asp Val Tyr
                930                 935                 940

atg atc atg gtc aag tgc tgg atg ata gac gca gat agt cgc cca aag          2880
Met Ile Met Val Lys Cys Trp Met Ile Asp Ala Asp Ser Arg Pro Lys
945                 950                 955                 960

ttc cgt gag ttg atc atc gaa ttc tcc aaa atg gcc cga gac ccc cag          2928
Phe Arg Glu Leu Ile Ile Glu Phe Ser Lys Met Ala Arg Asp Pro Gln
                965                 970                 975

cgc tac ctt gtc att cag ggg gat gaa aga atg cat ttg cca agt cct          2976
Arg Tyr Leu Val Ile Gln Gly Asp Glu Arg Met His Leu Pro Ser Pro
                980                 985                 990

aca gac tcc aac ttc tac cgt gcc  ctg atg gat gaa gaa  gac atg gac        3024
Thr Asp Ser Asn Phe Tyr Arg Ala  Leu Met Asp Glu Glu  Asp Met Asp
                995                 1000                1005

gac gtg  gtg gat gcc gac gag  tac ctc atc cca cag  cag ggc ttc          3069
Asp Val  Val Asp Ala Asp Glu  Tyr Leu Ile Pro Gln  Gln Gly Phe
        1010                1015                1020

ttc agc  agc ccc tcc acg tca  cgg act ccc ctc ctg  agc tct ctg          3114
Phe Ser  Ser Pro Ser Thr Ser  Arg Thr Pro Leu Leu  Ser Ser Leu
        1025                1030                1035

agt gca  acc agc aac aat tcc  acc gtg gct tgc att  gat aga aat          3159
Ser Ala  Thr Ser Asn Asn Ser  Thr Val Ala Cys Ile  Asp Arg Asn
        1040                1045                1050

ggg ctg  caa agc tgt ccc atc  aag gaa gac agc ttc  ttg cag cga          3204
```

```
Gly Leu Gln Ser Cys Pro Ile Lys Glu Asp Ser Phe Leu Gln Arg
    1055            1060            1065

tac agc tca gac ccc aca ggc gcc ttg act gag gac agc ata gac      3249
Tyr Ser Ser Asp Pro Thr Gly Ala Leu Thr Glu Asp Ser Ile Asp
    1070            1075            1080

gac acc ttc ctc cca gtg cct gaa tac ata aac cag tcc gtt ccc      3294
Asp Thr Phe Leu Pro Val Pro Glu Tyr Ile Asn Gln Ser Val Pro
    1085            1090            1095

aaa agg ccc gct ggc tct gtg cag aat cct gtc tat cac aat cag      3339
Lys Arg Pro Ala Gly Ser Val Gln Asn Pro Val Tyr His Asn Gln
    1100            1105            1110

cct ctg aac ccc gcg ccc agc aga gac cca cac tac cag gac ccc      3384
Pro Leu Asn Pro Ala Pro Ser Arg Asp Pro His Tyr Gln Asp Pro
    1115            1120            1125

cac agc act gca gtg ggc aac ccc gag tat ctc aac act gtc cag      3429
His Ser Thr Ala Val Gly Asn Pro Glu Tyr Leu Asn Thr Val Gln
    1130            1135            1140

ccc acc tgt gtc aac agc aca ttc gac agc cct gcc cac tgg gcc      3474
Pro Thr Cys Val Asn Ser Thr Phe Asp Ser Pro Ala His Trp Ala
    1145            1150            1155

cag aaa ggc agc cac caa att agc ctg gac aac cct gac tac cag      3519
Gln Lys Gly Ser His Gln Ile Ser Leu Asp Asn Pro Asp Tyr Gln
    1160            1165            1170

cag gac ttc ttt ccc aag gaa gcc aag cca aat ggc atc ttt aag      3564
Gln Asp Phe Phe Pro Lys Glu Ala Lys Pro Asn Gly Ile Phe Lys
    1175            1180            1185

ggc tcc aca gct gaa aat gca gaa tac cta agg gtc gcg cca caa      3609
Gly Ser Thr Ala Glu Asn Ala Glu Tyr Leu Arg Val Ala Pro Gln
    1190            1195            1200

agc agt gaa ttt att gga gca tga                                  3633
Ser Ser Glu Phe Ile Gly Ala
    1205            1210
```

<210> 10
<211> 1210
<212> PRT
<213> Homo sapiens

<400> 10

```
Met Arg Pro Ser Gly Thr Ala Gly Ala Ala Leu Leu Ala Leu Leu Ala
1               5                   10                  15

Ala Leu Cys Pro Ala Ser Arg Ala Leu Glu Glu Lys Lys Val Cys Gln
            20                  25                  30

Gly Thr Ser Asn Lys Leu Thr Gln Leu Gly Thr Phe Glu Asp His Phe
            35                  40                  45

Leu Ser Leu Gln Arg Met Phe Asn Asn Cys Glu Val Val Leu Gly Asn
        50                  55                  60
```

```
Leu Glu Ile Thr Tyr Val Gln Arg Asn Tyr Asp Leu Ser Phe Leu Lys
65              70              75              80

Thr Ile Gln Glu Val Ala Gly Tyr Val Leu Ile Ala Leu Asn Thr Val
                85              90              95

Glu Arg Ile Pro Leu Glu Asn Leu Gln Ile Ile Arg Gly Asn Met Tyr
            100             105             110

Tyr Glu Asn Ser Tyr Ala Leu Ala Val Leu Ser Asn Tyr Asp Ala Asn
        115             120             125

Lys Thr Gly Leu Lys Glu Leu Pro Met Arg Asn Leu Gln Glu Ile Leu
        130             135             140

His Gly Ala Val Arg Phe Ser Asn Asn Pro Ala Leu Cys Asn Val Glu
145             150             155             160

Ser Ile Gln Trp Arg Asp Ile Val Ser Ser Asp Phe Leu Ser Asn Met
            165             170             175

Ser Met Asp Phe Gln Asn His Leu Gly Ser Cys Gln Lys Cys Asp Pro
            180             185             190

Ser Cys Pro Asn Gly Ser Cys Trp Gly Ala Gly Glu Glu Asn Cys Gln
        195             200             205

Lys Leu Thr Lys Ile Ile Cys Ala Gln Gln Cys Ser Gly Arg Cys Arg
        210             215             220

Gly Lys Ser Pro Ser Asp Cys Cys His Asn Gln Cys Ala Ala Gly Cys
225             230             235             240

Thr Gly Pro Arg Glu Ser Asp Cys Leu Val Cys Arg Lys Phe Arg Asp
            245             250             255

Glu Ala Thr Cys Lys Asp Thr Cys Pro Pro Leu Met Leu Tyr Asn Pro
        260             265             270

Thr Thr Tyr Gln Met Asp Val Asn Pro Glu Gly Lys Tyr Ser Phe Gly
        275             280             285

Ala Thr Cys Val Lys Lys Cys Pro Arg Asn Tyr Val Val Thr Asp His
        290             295             300

Gly Ser Cys Val Arg Ala Cys Gly Ala Asp Ser Tyr Glu Met Glu Glu
305             310             315             320
```

33

```
Asp Gly Val Arg Lys Cys Lys Lys Cys Glu Gly Pro Cys Arg Lys Val
            325                 330                 335

Cys Asn Gly Ile Gly Ile Gly Glu Phe Lys Asp Ser Leu Ser Ile Asn
            340                 345                 350

Ala Thr Asn Ile Lys His Phe Lys Asn Cys Thr Ser Ile Ser Gly Asp
            355                 360                 365

Leu His Ile Leu Pro Val Ala Phe Arg Gly Asp Ser Phe Thr His Thr
370                 375                 380

Pro Pro Leu Asp Pro Gln Glu Leu Asp Ile Leu Lys Thr Val Lys Glu
385                 390                 395                 400

Ile Thr Gly Phe Leu Leu Ile Gln Ala Trp Pro Glu Asn Arg Thr Asp
                405                 410                 415

Leu His Ala Phe Glu Asn Leu Glu Ile Ile Arg Gly Arg Thr Lys Gln
            420                 425                 430

His Gly Gln Phe Ser Leu Ala Val Val Ser Leu Asn Ile Thr Ser Leu
            435                 440                 445

Gly Leu Arg Ser Leu Lys Glu Ile Ser Asp Gly Asp Val Ile Ile Ser
    450                 455                 460

Gly Asn Lys Asn Leu Cys Tyr Ala Asn Thr Ile Asn Trp Lys Lys Leu
465                 470                 475                 480

Phe Gly Thr Ser Gly Gln Lys Thr Lys Ile Ile Ser Asn Arg Gly Glu
                485                 490                 495

Asn Ser Cys Lys Ala Thr Gly Gln Val Cys His Ala Leu Cys Ser Pro
            500                 505                 510

Glu Gly Cys Trp Gly Pro Glu Pro Arg Asp Cys Val Ser Cys Arg Asn
            515                 520                 525

Val Ser Arg Gly Arg Glu Cys Val Asp Lys Cys Asn Leu Leu Glu Gly
            530                 535                 540

Glu Pro Arg Glu Phe Val Glu Asn Ser Glu Cys Ile Gln Cys His Pro
545                 550                 555                 560

Glu Cys Leu Pro Gln Ala Met Asn Ile Thr Cys Thr Gly Arg Gly Pro
                565                 570                 575
```

EP 2 592 141 B1

```
Asp Asn Cys Ile Gln Cys Ala His Tyr Ile Asp Gly Pro His Cys Val
        580         585             590

Lys Thr Cys Pro Ala Gly Val Met Gly Glu Asn Asn Thr Leu Val Trp
    595             600             605

Lys Tyr Ala Asp Ala Gly His Val Cys His Leu Cys His Pro Asn Cys
    610             615         620

Thr Tyr Gly Cys Thr Gly Pro Gly Leu Glu Gly Cys Pro Thr Asn Gly
625             630             635             640

Pro Lys Ile Pro Ser Ile Ala Thr Gly Met Val Gly Ala Leu Leu Leu
            645             650             655

Leu Leu Val Val Ala Leu Gly Ile Gly Leu Phe Met Arg Arg Arg His
            660             665             670

Ile Val Arg Lys Arg Thr Leu Arg Arg Leu Leu Gln Glu Arg Glu Leu
        675             680             685

Val Glu Pro Leu Thr Pro Ser Gly Glu Ala Pro Asn Gln Ala Leu Leu
    690             695             700

Arg Ile Leu Lys Glu Thr Glu Phe Lys Lys Ile Lys Val Leu Gly Ser
705             710             715             720

Gly Ala Phe Gly Thr Val Tyr Lys Gly Leu Trp Ile Pro Glu Gly Glu
            725             730             735

Lys Val Lys Ile Pro Val Ala Ile Lys Glu Leu Arg Glu Ala Thr Ser
            740             745             750

Pro Lys Ala Asn Lys Glu Ile Leu Asp Glu Ala Tyr Val Met Ala Ser
        755             760             765

Val Asp Asn Pro His Val Cys Arg Leu Leu Gly Ile Cys Leu Thr Ser
    770             775             780

Thr Val Gln Leu Ile Met Gln Leu Met Pro Phe Gly Cys Leu Leu Asp
785             790             795             800

Tyr Val Arg Glu His Lys Asp Asn Ile Gly Ser Gln Tyr Leu Leu Asn
            805             810             815

Trp Cys Val Gln Ile Ala Lys Gly Met Asn Tyr Leu Glu Asp Arg Arg
            820             825             830

Leu Val His Arg Asp Leu Ala Ala Arg Asn Val Leu Val Lys Thr Pro
```

835            840            845

Gln His Val Lys Ile Thr Asp Phe Gly Arg Ala Lys Leu Leu Gly Ala
850          855          860

Glu Glu Lys Glu Tyr His Ala Glu Gly Gly Lys Val Pro Ile Lys Trp
865          870          875          880

Met Ala Leu Glu Ser Ile Leu His Arg Ile Tyr Thr His Gln Ser Asp
885          890          895

Val Trp Ser Tyr Gly Val Thr Val Trp Glu Leu Met Thr Phe Gly Ser
900          905          910

Lys Pro Tyr Asp Gly Ile Pro Ala Ser Glu Ile Ser Ser Ile Leu Glu
915          920          925

Lys Gly Glu Arg Leu Pro Gln Pro Pro Ile Cys Thr Ile Asp Val Tyr
930          935          940

Met Ile Met Val Lys Cys Trp Met Ile Asp Ala Asp Ser Arg Pro Lys
945          950          955          960

Phe Arg Glu Leu Ile Ile Glu Phe Ser Lys Met Ala Arg Asp Pro Gln
965          970          975

Arg Tyr Leu Val Ile Gln Gly Asp Glu Arg Met His Leu Pro Ser Pro
980          985          990

Thr Asp Ser Asn Phe Tyr Arg Ala Leu Met Asp Glu Glu Asp Met Asp
995          1000          1005

Asp Val Val Asp Ala Asp Glu Tyr Leu Ile Pro Gln Gln Gly Phe
1010          1015          1020

Phe Ser Ser Pro Ser Thr Ser Arg Thr Pro Leu Leu Ser Ser Leu
1025          1030          1035

Ser Ala Thr Ser Asn Asn Ser Thr Val Ala Cys Ile Asp Arg Asn
1040          1045          1050

Gly Leu Gln Ser Cys Pro Ile Lys Glu Asp Ser Phe Leu Gln Arg
1055          1060          1065

Tyr Ser Ser Asp Pro Thr Gly Ala Leu Thr Glu Asp Ser Ile Asp
1070          1075          1080

Asp Thr Phe Leu Pro Val Pro Glu Tyr Ile Asn Gln Ser Val Pro
1085          1090          1095

```
Lys Arg  Pro Ala Gly Ser Val  Gln Asn Pro Val Tyr  His Asn Gln
    1100             1105             1110

Pro Leu  Asn Pro Ala Pro Ser  Arg Asp Pro His Tyr  Gln Asp Pro
    1115             1120             1125

His Ser  Thr Ala Val Gly Asn  Pro Glu Tyr Leu Asn  Thr Val Gln
    1130             1135             1140

Pro Thr  Cys Val Asn Ser Thr  Phe Asp Ser Pro Ala  His Trp Ala
    1145             1150             1155

Gln Lys  Gly Ser His Gln Ile  Ser Leu Asp Asn Pro  Asp Tyr Gln
    1160             1165             1170

Gln Asp  Phe Phe Pro Lys Glu  Ala Lys Pro Asn Gly  Ile Phe Lys
    1175             1180             1185

Gly Ser  Thr Ala Glu Asn Ala  Glu Tyr Leu Arg Val  Ala Pro Gln
    1190             1195             1200

Ser Ser  Glu Phe Ile Gly Ala
    1205             1210
```